# EUROPEAN PATENT APPLICATION

(11) **EP 1 111 046 A1**
(43) Date of publication of application: **27.06.2001**
(21) Application number: 99940628.3
(22) Date of filing: 02.09.1999
(51) Int. Cl.: C12N 15/12, C07K 14/47, C07K 16/18, A61K 49/00, A61K 39/395, A61K 45/00, A61K 31/70, G01N 33/50, G01N 33/15

(54) **NOVEL PROTEIN AND PROCESS FOR PRODUCING THE SAME**

(30) Priority: 03.09.1998 JP 25010898
(71) Applicant: Takeda Chemical Industries, Ltd., Osaka-shi, Osaka 541-0045 (JP)
(72) Inventor: ITOH, Yasuaki, Tsuchiura-shi, Ibaraki 300-0832 (JP); OGI, Kazuhiro, Tsukuba-shi, Ibaraki 305-0045 (JP); TANAKA, Hideyuki, Tsukuba-shi, Ibaraki 305-0821 (JP); KITADA, Chieko, Sakai-shi, Osaka 590-0073 (JP)
(74) Representative: Best, Michael, Dr.
(86) International application number: JP9904765
(87) International publication number: WO0014226

(57) **Abstract**

The present invention relates to a new secretory protein or a partial peptide thereof, or a salt thereof, DNA encoding for the protein, a recombinant vector comprising the DNA, a transformant, a method for producing the protein, a pharmaceutical preparation which comprises the protein or DNA, an antibody against the protein, a screening method/screening kit for a compound or a salt thereof promoting or inhibiting the activity of the protein, a compound obtained by the screening, and a pharmaceutical preparation which comprises the compound.

The protein of the present invention and the DNA encoding for the same can be used for a treating or preventing agent for various diseases such as immune diseases, pulmonary insufficiency, pancreatic insufficiency, infectious diseases or gastrointestinal disorders. In addition, the antibody against the protein of the present invention can be used for quantification of the protein of the present invention in a test solution. Further, the protein of the present invention is useful as a reagent for screening for a compound or a salt thereof promoting or inhibiting the activity of the protein of the present invention.

## Description

### Technical Field

The present invention relates to a novel secretory protein.

### Background Art

The living body performs balanced regulation of development, differentiation, growth and homeostasis through mutual information transfer among cells or tissues. In many cases, proteinous factors mediate such regulation. For example, many secretory factors (humoral factors) participating in the immune system and hematopoietic system are found and called cytokines. These contain lymphokines, monokines, interferons, colony-stimulating factors and tumor necrosis factors.

The relationship thereof with diseases or a method of utilizing these factors as pharmaceutical preparations is extensively studied. Further, humoral factors such as peptide hormones and growth factors produced by internal-secretion tissues also play a very important role in homeostasis and growth. Such factors are also extensively studied for use as pharmaceutical preparations. These proteinous factors important for the living body have been found with their biological activity as an indicator. Other proteinous factors have also been found on the basis of their homology as an indicator to known biologically active proteins. It is estimated that besides those previously found, a large number of unknown proteinous factors having biological activity occur to permit complex living things particularly mammals to be healthy. In recent years, a vast number of novel genes have been found through e.g. large-scaled sequencing of cDNA library, but the information on their sequences is fragmentary and incorrect, thus making it difficult to select absolutely new useful gene products therefrom. Novel gene products, which were found in the information on DNA sequences with the help of information processing technology by computer, are now attempted to be utilized in biology, medicine, veterinary medicine etc. (Trends in Biotechnology, Vol. 14, pp. 294-298, 1996). However, there is still a need for not only information processing technology but also more detailed analysis and experiments in order to find truly useful products.

### Disclosure of Invention

The object of the present invention is to provide a novel protein, its partial peptide, or their salts, a recombinant vector, a transformant, a process for producing said protein, a pharmaceutical preparation which comprises said protein or partial peptide, and an antibody against said protein etc., which are applicable in biology, medicine and veterinary medicine.

As a result of their eager study for solving the problem described above, the present inventors successfully found cDNA having a novel base sequence at high levels in the lungs, trachea, stomach etc., and found that a protein encoded by said cDNA is a humoral factor actually secreted extracellularly. As a result of further examination on the basis of these findings, the present inventors arrived at the present invention. That is, the present invention provides:
1. A protein which comprises an amino acid sequence identical or substantially identical with the amino acid sequence represented by SEQ ID NO: 1, SEQ ID NO: 2 or SEQ ID NO: 3, or a salt thereof;
2. A partial peptide of the protein of the above item 1, or a salt thereof;
3. A method for producing the protein of the above item 1 or the partial peptide of the above item 2, or a salt thereof, which comprises culturing a transformant transformed with a recombinant vector comprising DNA encoding for the protein of the above item 1 or the partial peptide of the above item 2 and forming the protein or the partial peptide;
4. An antibody against the protein of the above item 1 or the partial peptide of the above item 2, or a salt thereof;
5. A method of screening for a compound or a salt thereof promoting or inhibiting the activity of the protein of the above item 1 or the partial peptide of the above item 2, or a salt thereof, which comprises use of the protein of the above item 1 or the partial peptide of the above item 2, or a salt thereof;
6. A kit for screening for a compound or a salt thereof promoting or inhibiting the activity of the protein of the above item 1 or the partial peptide of the above item 2, or a salt thereof, which comprises the protein of the above item 1 or the partial peptide of the above item 2, or a salt thereof;
7. A compound or a salt thereof promoting or inhibiting the activity of the protein of the above item 1 or the partial peptide of the above item 2, or a salt thereof, which can be obtained by use of the method of screening of the above item 5 or the kit for screening of the above item 6;
8. A pharmaceutical preparation which comprises a compound or a salt thereof promoting or inhibiting the activity of the protein of the above item 1 or the partial peptide of the above item 2, or a salt thereof, which can be obtained by use of the method of screening of the above item 5 or the kit for screening of the above item 6;
9. A diagnostic agent which comprises the antibody of the above item 4; and
10. A pharmaceutical preparation which comprises the antibody of the above item 4.

Further, the present invention provides:
11. The protein according to item 1, wherein the amino acid sequence substantially identical with the amino acid sequence represented by SEQ ID NO: 1, SEQ ID NO: 2 or SEQ ID NO: 3 is an amino acid sequence having about 50% or more (preferably about 60% or more, more preferably 70% or more, more preferably about 80% or more, particularly preferably about 90% or more, and most preferably about 95% or more) homology to the amino acid sequence represented by SEQ ID NO: 1, SEQ ID NO: 2 or SEQ ID NO: 3;
12. The protein according to item 1, wherein the amino acid sequence substantially identical with the amino acid sequence represented by SEQ ID NO: 1, SEQ ID NO: 2 or SEQ ID NO: 3 is (1) the amino acid sequence represented by SEQ ID NO: 1, SEQ ID NO: 2 or SEQ ID NO: 3 with a deletion of one or more (preferably 1 to about 30) amino acids, (2) the amino acid sequence represented by SEQ ID NO: 1, SEQ ID NO: 2 or SEQ ID NO: 3 with an addition of one or more (preferably 1 to about 30) amino acids, (3) the amino acid sequence represented by SEQ ID NO: 1, SEQ ID NO: 2 or SEQ ID NO: 3 with a substitution of one or more (preferably 1 to about 30) amino acids by other amino acids, or (4) a combined amino acid sequence of the above;
13. The peptide according to item 2, which comprises an amino acid sequence having the amino acid sequence at the 23 to 119 positions in the amino acid sequence represented by SEQ ID NO: 1, SEQ ID NO: 2 or SEQ ID NO: 3;
14. A recombinant vector which comprises DNA encoding for the protein of the above item 1 or the partial peptide of the above item 2;
15. A transformant transformed with the recombinant vector of the above item 14; and
16. The pharmaceutical preparation according to the above item 10, which is a treating or preventing agent for immune diseases, respiratory diseases such as pulmonary insufficiency, pancreatic insufficiency, infectious diseases, or digestive diseases such as gastrointestinal disorders.

Further, the present invention is applicable not only to fundamental study such as molecular-weight marker, tissue maker, chromosome mapping, identification of hereditary diseases, design of primer and probe, but also for therapeutic or preventive purposes in fields such as inhibition of cancer metastasis, detection of cancer metastasis, regulation of cellular differentiation and multiplication, induction of cytokines, regulation of new formation of blood vessels, regulation of hematopoiesis, regulation of blood coagulation, infectious diseases, metabolic regulation, treatment of wounds and burns, anti-inflammation, gene therapy etc. In addition, the present invention is applicable for therapeutic or preventive purposes against diseases such as trachea- and bronchus-related diseases (e.g., bronchitis, influenza infectious diseases, bronchial asthma, upper trachea inflammation, bronchiectasis etc.), lung-related diseases (lung cancer, tuberculosis, pneumonia, pulmonary emphysema, pulmonary infarct, pulmonary stagnation, respiratory insufficiency, cystic pulmonary fibroma, pulmonary sarcoidosis, pulmonary edema, pulmonary high blood pressure, pneumoconiosis etc.), stomach-related diseases (e.g., *Helicobacter pylori* infectious disease, digestive ulcer, gastric cancer, stomach atony, gastritis, Mallory-Weiss syndrome, stomach expansion, abnormal gastric juice secretion, Menetrier's disease (gigantic hypertrophic gastritis), stomach tetany, torso disease (stomach dizziness), gastrointestinal neurosis), diabetes, digestive insufficiency, maintenance of enteric bacteria, etc.

### Brief Description of Drawings

Fig. 1 shows the base sequence of DNA encoding for the protein of the present invention obtained in Example 1, as well as an amino acid sequence deduced from said base sequence.

Fig. 2 shows the base sequence of rat TGC-440 obtained in Example 3, as well as an amino acid sequence deduced from said base sequence.

Fig. 3 shows the base sequence of mouse TGC-440 obtained in Example 4, as well as an amino acid sequence deduced from said base sequence.

Fig. 4 shows a restriction enzyme map of the animal cell expression vector pCAN618 obtained in the Reference Example.

Fig. 5 shows the result of Western blotting performed in Example 7, wherein:
Control: Culture supernatant (using Opti-MEM medium containing 0.25 mMpABSF and 0.05% CHAPS) of COS7 cells transfected with pCAN618;
Lane 1: Culture supernatant of the cells transfected with pCAN618/huTGC440, where Opti-MEM medium was used;
Lane 2: Culture supernatant of the cells transfected with pCAN618/huTGC440, where Opti-MEM medium containing 0.25 mM pABSF was used;
Lane 3: Culture supernatant of the cells transfected with pCAN618/huTGC440, where Opti-MEM medium containing 0.05% CHAPS was used; and
Lane 4: Culture supernatant of the cells transfected with pCAN618/huTGC440, where Opti-MEM medium containing 0.25 mM pABSF and 0.05% CHAPS was used.

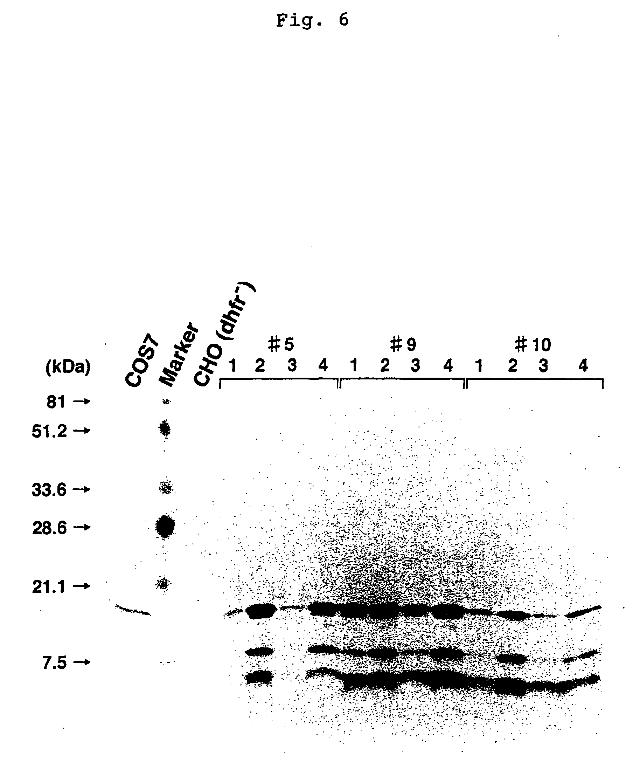

Fig. 6 shows the result of Western blotting performed in Example 8, wherein:
COS7: Culture supernatant (using Opti-MEM medium containing 0.25 mM pABSF and 0.05% CHAPS) of COS7 cells transfected with pDRL440H;
Marker: Molecular marker;
#5: #5 clone;
#9: #9 clone;
#10: #10 clone;
CHO(dhfr⁻): Culture supernatant (using αMEM medium containing 0.25 mM pABSF and 0.05% CHAPS) of CHO(dhfr⁻) cells not transfected;
Lane 1: Culture supernatant of the cells transfected with pDRL440H, where αMEM medium was used;
Lane 2: Culture supernatant of the cells transfected with pDRL440H, where αMEM medium containing 0.25 mM pABSF was used;
Lane 3: Culture supernatant of the cells transfected with pDRL440H, where αMEM medium containing 0.05% CHAPS was used; and
Lane 4: Culture supernatant of the cells transfected with pDRL440H, where αMEM medium containing 0.25 mM pABSF and 0.05% CHAPS was used.

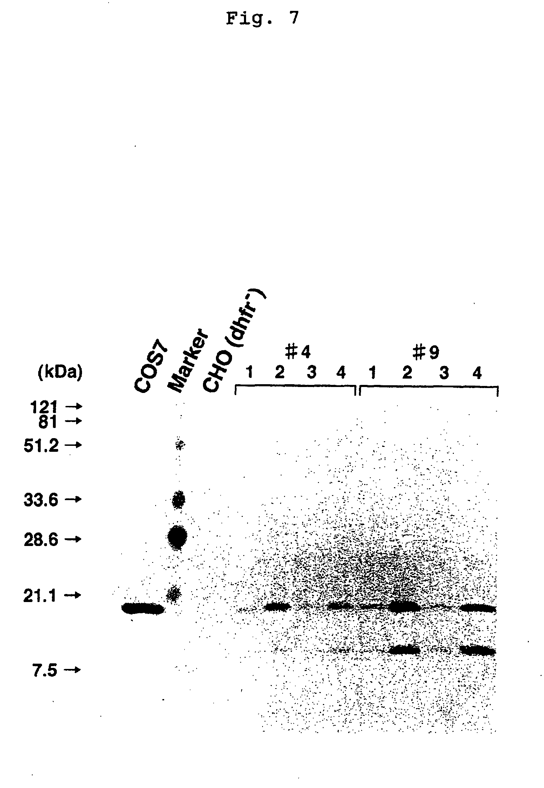

Fig. 7 shows the result of Western blotting performed in Example 8, wherein:
COS7: Culture supernatant (using Opti-MEM medium containing 0.25 mM pABSF and 0.05% CHAPS) of COS7 cells transfected with pDRL440R;
Marker: Molecular marker;
#4: #4 clone;
#9: #9 clone;
CHO(dhfr⁻): Culture supernatant (using αMEM medium containing 0.25 mM pABSF and 0.05% CHAPS) of CHO(dhfr⁻) cells not transfected;
Lane 1: Culture supernatant of the cells transfected with pDRL440R, where αMEM medium was used;
Lane 2: Culture supernatant of the cells transfected with pDRL440R, where αMEM medium containing 0.25 mM pABSF was used;
Lane 3: Culture supernatant of the cells transfected with pDRL440R, where αMEM medium containing 0.05% CHAPS was used; and
Lane 4: Culture supernatant of the cells transfected with pDRL440R, where αMEM medium containing 0.25 mM pABSF and 0.05% CHAPS was used.

### Best Mode for Carrying Out the Invention

The protein which comprises an amino acid sequence identical or substantially identical with the amino acid sequence represented by SEQ ID NO: 1, SEQ ID NO: 2 or SEQ ID NO: 3 in the present invention (referred to hereinafter as the protein of the present invention) may be a protein derived from cells (e.g., hepatic cells, spleen cells, nerve cells, glia cells, pancreatic β-cells, bone marrow cells, mesangial cells, Langerhans' cells, epidermal cells, epithelial cells, endothelial cells, fibroblasts, fibrous cells, muscular cells, fat cells, immune cells (e.g., macrophage, T cell, B cell, natural killer cell, mast cell, neutrophils, basophils, eosinophils, monocyte), megakaryocyte, synovial membrane cells, soft-bone cells, bone cells, osteoblast, osteoclast, mammary gland cells or interstitial cells, or their precursor cells, stem cells or cancer cells) or any tissues having such cells, for example, brain and each site of brain (e.g., olfactory bulb, tonsil nuclei, cerebral basal bulb, hippocampus, thalamus, hypothalamus, cerebral cortex, medulla bulb, cerebellum), spinal cord, pituitary, stomach, pancreas, kidney, liver, gonads, thyroid glands, galls, bone marrow, adrenals, skin, muscles, lungs, digestive tracts (e.g., large and small intestines), blood vessels, heart, thymus, spleen, salivary glands, peripheral blood, prostate, testicles, ovary, placenta, uterus, bone, cartilage, joints, skeletal muscles from humans or warm-blooded animals (e.g., guinea pig, rat, mouse, chicken, rabbit, pig, sheep, cow, monkey etc.), as well as a recombinant protein or a synthetic protein.

The amino acid sequence substantially identical with the amino acid sequence represented by SEQ ID NO: 1, SEQ ID NO: 2 or SEQ ID NO: 3 includes an amino acid sequence having about 50% or more, preferably about 60% or more, further preferably 70% or more, more preferably about 80% or more, particularly preferably about 90% or more, and most preferably about 95% or more homology to the amino acid sequence represented by SEQ ID NO: 1, SEQ ID NO: 2 or SEQ ID NO: 3.

Usually, the protein of the present invention has a signal peptide, and thus the protein can be secreted efficiently extracellularly.

The protein of the present invention having an amino acid sequence substantially identical with the amino acid sequence represented by SEQ ID NO : 1, SEQ ID NO : 2 or SEQ ID NO : 3 is preferably. for example, a protein having an amino acid sequence substantially identical with the amino acid sequence represented by SEQ ID NO: 1, SEQ ID NO: 2 or SEQ ID NO: 3 and having substantially homogeneous properties with those of a protein having the amino acid sequence represented by SEQ ID NO: 1, SEQ ID NO: 2 or SEQ ID NO: 3.

The substantially homogeneous properties indicate, for example, that the protein in question is secreted and acts as a humoral factor. The terms "substantially homogeneous" mean that between those proteins in question, their properties are qualitatively homogeneous. It follows that between those proteins in question, their qualitative properties such as secretion and solubility are preferably identical (e.g., about 0.1- to 10-fold, preferably about 0.5- to 10-fold, and more preferably 0.5- to 2-fold), while their quantitative properties such as molecular weight may be different.

The protein of the present invention includes, for example, proteins which comprises the amino acid sequence substantially identical with the amino acid sequence represented by SEQ ID NO: 1, SEQ ID NO: 2 or SEQ ID NO: 3 is (1) the amino acid sequence represented by SEQ ID NO: 1, SEQ ID NO: 2 or SEQ ID NO: 3 with a deletion of one or more (preferably 1 to about 30, more preferably 1 to about 10, and most preferably 1 to 5) amino acids, (2) the amino acid sequence represented by SEQ ID NO: 1, SEQ ID NO: 2 or SEQ ID NO: 3 with an addition of one or more (preferably 1 to about 30, more preferably 1 to about 10, and most preferably 1 to 5) amino acids, (3) the amino acid sequence represented by SEQ ID NO: 1, SEQ ID NO: 2 or SEQ ID NO: 3 with an insertion of one or more (preferably 1 to about 30, more preferably 1 to about 10, and most preferably 1 to 5) amino acids, (4) the amino acid sequence represented by SEQ ID NO: 1, SEQ ID NO: 2 or SEQ ID NO: 3 with a substitution of one or more (preferably 1 to about 30, more preferably 1 to about 10, and most preferably 1 to 5) amino acids by other amino acids, and (5) a combination of these amino acid sequences, or a protein called mutein comprising such a combination of these amino acid sequences.

If an amino acid sequence has an insertion, deletion or substitution as described above, the position of the insertion, deletion or substitution includes, but is not limited to, aposition outside of those common amino acid sequences in the amino acid sequences represented by SEQ ID NO: 1, SEQ ID NO: 2 and SEQ ID NO: 3.

In the present specification, the N-terminal (amino terminal) of protein is placed in the left and the C-terminal (carboxyl terminal) in the right in accordance with a peptide notation system. The protein of the present invention, including a protein having the amino acid sequence represented by SEQ ID NO: 1, usually has a carboxyl group (-COOH) or carboxylate (-COO⁻) at the C-terminal, but may have an amide (-CONH₂) or ester (-COOR) at the C-terminal.

The group R in this ester includes, for example, C₁₋₆ alkyl groups such as methyl, ethyl, n-propyl, isopropyl and n-butyl, C₃₋₈ cycloalkyl groups such as cyclopentyl and cyclohexyl, C₆₋₁₂ aryl groups such as phenyl and α-naphthyl, and C₇₋₁₄ aralkyl groups, for example, phenyl-C₁₋₂ alkyl groups such as benzyl and phenetyl and α-naphthyl-C₁₋₂ alkyl groups such as α-naphthylmethyl, as well as a pivaloyloxymethyl group used generally as oral ester.

If the protein of the present invention has a carboxyl group (or carboxylate) at other position than the C-terminal, the protein may have said carboxyl group amidated or esterified as the protein of the present invention. The ester used in this case includes, for example, the ester at the C-terminal as described above.

Also, the protein of the present invention includes proteins wherein an amino group of an N-terminal amino acid residue (e.g. methionine residue) is protected with a protecting group (e.g., C₁₋₆ acyl group including C₁₋₆ alkanoyl group such as formyl group and acetyl group), an N-terminal glutamic residue formed in vivo by cleavage is oxidized into pyroglutamine, or a substituent group (e.g., -OH, -SH, amino group, imidazole group, indole group, guanidino group or the like) on a side chain of an intramolecular amino acid is protected with a suitable protecting group (e.g., C₁₋₆ acyl group including C₁₋₆ alkanoyl group such as formyl group and acetyl group), as well as conjugated proteins such as glycoprotein having sugar chains bound thereto.

Examples of the protein of the present invention include, for example, a human-derived protein having the amino acid sequence represented by SEQ ID 1, a rat-derived protein having the amino acid sequence represented by SEQ ID 2, and a mouse-derived protein having the amino acid sequence represented by SEQ ID 3.

The partial peptide of the protein of the present invention may be any partial peptide derived from the protein of the present invention described above and preferably having similar properties to those of the protein of the present invention described above. Such partial peptide includes, for example, peptides having at least 5, preferably 20 or more, further preferably 30 or more, more preferably 50 or more, and most preferably 80 or more amino-acids sequence from the amino acid sequence constituting the protein of the present invention.

Among others, preferably used are peptides having an amino acid sequence identical or substantially identical with the amino acid sequence at the 23 to 119 positions in the amino acid sequence represented by SEQ ID NO: 1, SEQ ID NO: 2 or SEQ ID NO: 3 and having substantially homogeneous properties with those of the protein of the present invention.

As used herein, the terms "substantially homogeneous properties" have the same meanings as defined above.

Also, the partial peptide of the present invention may have deletion of one or more (preferably 1 to about 10 and more preferably several (1 to 5)) amino acids, addition of one or more (preferably 1 to about 20, more preferably 1 to about 10 and most preferably several (1 to 5)) amino acids, insertion of one or more (preferably 1 to about 20, more preferably 1 to about 10 and most preferably several (1 to 5)) amino acids, or substitution of one or more (preferably 1 to about 10, more preferably several, and most preferably 1 to about 5) amino acids by other amino acids.

The partial peptide of the present invention usually has a carboxyl group (-COOH) or carboxylate (-COO⁻) at the C-terminal, but may have an amide (-CONH₂) or ester (-COOR) at the C-terminal.

Also, the partial peptide of the present invention includes peptides wherein an amino group of an N-terminal amino acid residue (e.g. methionine residue) is protected with a protecting group, an N-terminal glutamic residue formed in vivo by cleavage is oxidized into pyroglutamine, or a substituent group on a side chain of an intramolecular amino acid is protected with a suitable protecting group, as well as conjugated peptides such as glycopeptide having sugar chains bound thereto.

Because the partial peptide of the present invention can be used as an antigen for raising an antibody, it is not always necessary for the partial peptide to have the activity possessed by the protein of the present invention.

Examples of salts of the protein or partial peptide of the present invention include salts with physiologically acceptable acids (e.g., inorganic acids, organic acids) or bases (e.g., alkali metal salts), and preferably physiologically acceptable acid addition salts. Such salts include, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The protein or partial peptide of the present invention or a salt thereof can be produced by per se known protein purification techniques from the human or warm-blooded animal cells or tissues described above, or by culturing transformants comprising DNA encoding for the protein as described below. Alternatively, these can also be produced by the peptide synthetic method described below.

For production from human or mammalian tissues or cells, the human or mammalian tissues or cells are homogenized and then extracted with e.g. an acid, and the extract can be subjected to purification and isolation by a combination of chromatographic techniques such as reverse-phase chromatography, ion-exchange chromatography etc.

For synthesis of the protein or partial peptide of the present invention or a salt thereof or amide derivatives thereof, usually commercially available resin for protein synthesis can be used. Such resin includes, for example, chloromethyl resin, hydroxymethyl resin, benzhydryl amine resin, aminomethyl resin, 4-benzyloxybenzyl alcohol resin, 4-methylbenzhydryl amine resin, PAM resin, 4-hydroxymethylmethylphenylacetamidemethyl resin, polyacrylamide resin, 4-(2',4'-dimethoxyphenyl-hydroxymethyl) phenoxy resin, 4-(2',4'-dimethoxyphenyl-Fmoc aminoethyl)phenoxy resin, and so forth. On the resin described above, each amino acid with the α-amino group and side-chain functional group properly protected is condensed sequentially in accordance with the sequence of the desired protein by the per se known condensation methods. At the end of the reaction, the protein is cleaved off from the resin while various protecting groups are removed, and the product is subjected to a reaction of forming intramolecular disulfide bonds in a highly dilute solution to give the desired protein or an amide thereof.

A wide variety of activating reagents usable for protein synthesis can be used for condensation of the protected amino acids described above, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids along with racemization inhibitors (e.g., HOBt, HOOBt) can be added to the resin directly or after the protected amino acids were previously activated as symmetric acid anhydrides or HOBt esters or HOOBt esters.

The solvent used for activation of each protected amino acid or for condensation thereof with the resin can be selected as necessary from those solvents known to be usable in protein condensation reaction. Examples of such solvent include acid amides such as N,N-dimethylformamide, N,N-dimethylacetamide and N-methylpyrrolidone, halogenated hydrocarbons such as methylene chloride and chloroform, alcohols such as trifluoroethanol, sulfoxides such as dimethyl sulfoxide, ethers such as pyridine, dioxane and tetrahydrofuran, nitriles such as acetonitrile and propionitrile, esters such as methyl acetate and ethyl acetate, or a suitable mixture thereof. The reaction temperature is usually selected as necessary within the range known to be usable in the reaction of forming protein bonds, and usually the reaction temperature is selected within the range of about -20 °C to 50 °C. The activated amino acid derivatives are used usually in excess (1.5- to 4-fold). When their condensation is insufficient, their sufficient condensation is achieved as a result of a test using ninhydrin reaction by repeatedly carrying out the condensation reaction without conducting elimination of the protecting groups. When their sufficient condensation is not achieved even by repeatedly carrying out the reaction, the unreacted amino acids are acetylated with acetic anhydride or acetyl imidazole so that the subsequent reaction cannot be influenced.

The protecting groups for amino groups in the starting materials include, for example, Z, Boc, t-pentyloxycarbonyl, isobornyloxycarbonyl, 4-methoxybenzyloxycarbonyl, C1-Z, Br-Z, adamantyloxycarbonyl, trifluoroacetyl, phthaloyl, formyl, 2-nitrophenylsulphenyl, diphenylphosfinothioyl, Fmoc etc.

The carboxyl group can be protected by, for example, alkyl esterification (e.g., straight-chain, branched or cyclic alkyl esterification such as methyl, ethyl, propyl, butyl, t-butyl, cyclopentyl, cyclohexyl, cycloheptyl, cycloctyl or 2-adamantyl esterification), aralkyl esterification (e.g., benzyl esterification, 4-nitrobenzyl esterification, 4-methoxybenzyl esterification, 4-chlorobenzyl esterification, benzhydryl esterification), phenacyl esterification, benzyloxycarbonylhydrazidation, t-butoxycarbonylhydrazidation, tritylhydrazidation etc.

The hydroxyl group in serine can be protected by, for example, esterification or etherification. A suitable group used in this esterification includes, for example, lower (C₁₋₆) alkanoyl groups such as acetyl group, alloyl groups such as benzoyl group, and carbonic acid-derived groups such as benzyloxycarbonyl group and ethoxycarbonyl group. A suitable group for etherification includes, for example, a benzyl group, tetrahydropyranyl group, t-butyl group etc.

The protecting group used for the phenolic hydroxyl group in tyrosine includes, for example, Bzl, Cl₂-Bzl, 2-nitrobenzyl, Br-Z, t-butyl etc.

The protecting group used for imidazole in histidine includes, for example, Tos, 4-methoxy-2,3,6-trimethylbenzenesulfonyl, DNP, benzyloxymethyl, Bum, Boc, Trt, Fmoc etc.

Activated carboxyl groups in the starting materials include, for example, the corresponding acid anhydrides, azides and active esters (i.e. esters with alcohols such as pentachlorophenol, 2,4,5-trichlorophenol, 2 ,4-dinitrophenol, cyanomethyl alcohol, p-nitrophenol, HONB, N-hydroxysuccinamide, N-hydroxyphthalimide and HOBt). The activated amino groups in the starting materials include, for example, the corresponding phosphoric acid amides.

Examples of methods for removing (leaving) of the protecting groups include catalytic reduction in a hydrogen stream in the presence of a catalyst such as Pd-black or Pd-carbon, acid treatment using anhydrous hydrogen fluoride, methane sulfonic acid, trifluoromethane sulfonic acid, trifluoroacetic acid or a mixed solution thereof, base treatment using diisopropylethylamine, triethylamine, piperidine or piperazine, and reduction using sodium in liquid ammonia. The leaving reaction by the acid treatment described above is carried out generally at a temperature of about -20 °C to 40 °C, and it is effective in the acid treatment to add a cation scavenger such as anisole, phenol, thioanisole, m-cresol, p-cresol, dimethylsulfide, 1,4-butanedithiol and 1,2-ethanedithiol. A 2,4-dinitrophenyl group used as a protecting group for imidazole in histidine can also be removed by treatment with thiophenol, while a formyl group used as a protecting group for indole in tryptophan can be removed not only by deprotection by acid treatment in the presence of 1,2-ethanedithiol or 1,4-butanedithiol, but also by alkali treatment using dilute sodium hydroxide solution or dilute ammonia.

Protection and protecting groups for functional groups which should not participate in the reaction of the starting materials, elimination of the protecting groups, and activation of functional groups participating in the reaction can be selected as necessary from known groups or known means.

Another method of obtaining an amide derivative of the protein includes, for example, protecting the α-carboxyl group of a C-terminal amino acid by amidation, then extending a peptide (protein) chain at the side of the amino group until it attains desired chain length, and thereafter producing a protein of said peptide chain from which only the protecting group for the N-terminal α-amino group was removed and a protein of said peptide chain from which only the protecting group for the C-terminal carboxyl group was removed, followed by condensation both the proteins in the mixed solvent as described above. The details of the condensation reaction are the same as described above. The protected protein obtained by condensation is purified, and every protecting group is removed by the method descried above, whereby the desired crude protein can be obtained. This crude protein is purified by a wide variety of known purification techniques, and by lyophilizing its major fraction, the desired amide derivative of the protein can be obtained.

To obtain an ester derivative of the protein, for example the α-carboxyl group of a C-terminal amino acid is condensed with desired alcohol to form an amino acid ester from which the desired ester derivative of the protein can be obtained in the same manner as for the amide derivative of the protein.

The partial peptide of the present invention or a salt thereof can be produced according to a peptide synthesis method known per se or by cleaving the protein of the present invention with a suitable peptidase. For example, the peptide synthesis method may be the solid- or liquid-phase synthesis method. That is, the desired peptide can be obtained by condensation of a partial peptide or amino acids capable of constituting the partial peptide of the present invention with the remainder, followed by elimination of protecting groups if any from the product. As the known condensation method and the elimination of the protecting groups, mention is made of e.g. the methods described in (1) to (5) below:
(1) M. Bodanszky and M. A. Ondetti, Peptide Synthesis, Interscience Publisher, New York (1966);
(2) Schroeder and Luebke, The Peptide, Academic Press, New York (1965);
(3) Nobuo Izumiya et al., Basis and Experiments in Peptide Synthesis (in Japanese), Maruzen Co., Ltd. (1975);
(4) Haruaki Yajima and Shunpei Sakakibara, Biochemical Experimental Course 1, Protein Chemistry IV, 205, (1977); and
(5) Haruaki Yajima (supervisor), Development of medicines, a second series, vol.14, Peptide Synthesis, Hirokawashoten.

After the reaction, the partial peptide of the present invention can be isolated and purified by a combination of conventional purification techniques such as solvent extraction, distillation, column chromatography, liquid chromatography and recrystallization. If the partial peptide is obtained in a free form by these methods, the product can be converted into a suitable salt by a known method or its analogous method, or if the partial peptide is obtained in a salt form, it can be converted into a free peptide or other salts by a known method or its analogous method.

The DNA encoding for the protein of the present invention may be any DNA which comprises a base sequence enencoding for the protein of the present invention described above. This DNA may also be genomic DNA, cDNA derived form the above-described cells or tissues, or synthetic DNA.

The vector used in the library may be bacteriophage, plasmid, cosmid, phagimide or the like. A total RNA or mRNA fraction prepared from the cells and tissues described above can also be used in direct amplification by Reverse Transcriptase Polymerase Chain Reaction (abbreviated hereinafter to RT-PCR method).

The DNA encoding for the protein of the present invention includes, for example, a DNA having (1) a DNA comprising the base sequence represented by SEQ ID NO: 4 or a DNA having a base sequence which hybridizes under high stringent conditions with the base sequence represented by SEQ ID NO: 4 and encoding for a protein having substantially homogeneous properties (e.g., secretory action) with those of the protein of the present invention, (2) a DNA comprising the base sequence represented by SEQ ID NO: 5 or a DNA having a base sequence which hybridizes under high stringent conditions with the base sequence represented by SEQ ID NO: 5 and encoding for a protein having substantially homogeneous properties with those of the protein of the present invention, or (3) a DNA comprising the base sequence represented by SEQ ID NO: 6 or having a base sequence which hybridizes under high stringent conditions with the base sequence represented by SEQ ID NO: 6 and encoding for a protein having substantially homogeneous properties with those of the protein of the present invention, and encoding for a protein having substantially homogeneous properties with those of the protein of the present invention.

The DNA capable of hybridizing under high stringent conditions with the base sequence represented by any one of SEQ ID NO: 4 to SEQ ID NO: 6 includes, for example, DNA having a base sequence having about 60% or more, preferably about 70% or more, and more preferably 80% or more homology to the amino acid sequence represented by any one of SEQ ID NO: 4 to SEQ ID NO: 6.

Hybridization can be carried out according to a per se known method or its analogous method, for example a method described in Molecular Cloning, 2nd ed. (J. Sambrook et al., Cold Spring Harbor Lab. Press, 1989). If a commercial library is used, hybridization can be carried out according to the manufacture's instructions. Preferably, hybridization can be conducted under high stringent conditions.

The high stringent conditions refer to those conditions under which the concentration of sodium is about 19 to 40 mM, preferably about 19 to 20 mM, and the temperature is about 50 to 70 °C, preferably about 60 to 65 °C.

Specifically, the DNA encoding for a protein having the amino acid sequence represented by SEQ ID NO: 1 includes a DNA having the base sequence represented by SEQ ID NO: 4; the DNA encoding for a protein having the amino acid sequence represented by SEQ ID NO: 2 includes a DNA having the base sequence represented by SEQ ID NO: 5; and the DNA encoding for a protein having the amino acid sequence represented by SEQ ID NO: 3 includes a DNA having the base sequence represented by SEQ ID NO: 6.

The DNA encoding for the partial peptide of the present invention may be any DNA comprising a base sequence encoding for the above-described partial peptide of the present invention. It may also be genomic DNA, cDNA derived the above cells or tissues, or synthetic DNA.

The DNA encoding for the partial peptide of the present invention includes, for example, (1) a DNA having a partial base sequence of DNA having the base sequence represented by SEQ ID NO: 4, a DNA having DNA having the base sequence represented by SEQ ID NO: 10 or a DNA having a base sequence which hybridizes under high stringent conditions with the base sequence represented by SEQ ID NO: 10 and having a partial base sequence of DNA encoding for a protein having substantially homogeneous properties with those of the protein of the present invention, (2) a DNA having a partial base sequence of DNA having the base sequence represented by SEQ ID NO: 5 or a DNA having the base sequence represented by SEQ ID NO: 11 or a DNA having a base sequence which hybridizes under high stringent conditions with the base sequence represented by SEQ ID NO: 11 and having a partial base sequence of DNA encoding for a protein having substantially homogeneous properties with those of the protein of the present invention, and (3) a DNA having a partial base sequence of DNA having the base sequence represented by SEQ ID NO: 6,a DNA having the base sequence represented by SEQ ID NO: 12 or a DNA having a base sequence which hybridizes under high stringent conditions with the base sequence represented by SEQ ID NO: 12 and having a partial base sequence of DNA encoding for a protein having substantially homogeneous properties with those of the protein of the present invention.

The DNA capable of hybridizing under high stringent conditions with the base sequence represented by any one of SEQ ID NO: 10 to SEQ ID NO: 12 has the same meanings as defined above.

The hybridization method and high stringent conditions are those described above.

Specifically, the DNA encoding for a partial peptide having the amino acid sequence represented by SEQ ID NO: 7 includes a DNA having the base sequence represented by SEQ ID NO: 10; the DNA encoding for a protein having the amino acid sequence represented by SEQ ID NO: 8 includes a DNA having the base sequence represented by SEQ ID NO: 11; and the DNA encoding for a protein having the amino acid sequence represented by SEQ ID NO: 9 includes a DNA having the base sequence represented by SEQ ID NO: 12.

As a means of cloning the DNA encoding completely for the protein and partial peptide of the present invention (these proteins are referred to collectively as the protein of the present invention in the following description of cloning and expression of the DNA encoding for these proteins), it is possible to use amplification by the PCR method using synthetic DNA primers having a partial base sequence of the protein of the present invention as well as selection by hybridization of the DNA integrated in a suitable vector with a labeled DNA fragment or synthetic DNA encoding for a part or the whole of the protein of the present invention. Hybridization can be carried out according to a method described in, for example, Molecular Cloning, 2nd ed. (J. Sambrook et al., Cold Spring Harbor Lab. Press, 1989). If a commercial library is used, hybridization can be carried out according to the manufacture's instructions.

Conversion of DNA into a base sequence can be carried out by a per se known method, for example by the Gupped duplex method or Kunkel method or its analogous method by using PCR or a known kit such as Mutant™-G (Takara Shuzo Co., Ltd.) or Mutant™-K (Takara Shuzo Co., Ltd.).

A DNA encoding a cloned protein can be used as it is or, if desired, by digesting with a restriction enzyme or adding a linker thereto. The DNA may have ATG as a translation initiation codon at the 5'-terminal thereof and TAA, TGA or TAG as a translation termination codon at the 3'-terminal thereof. These translation initiation and termination codons can also be added to the DNA via a suitable synthetic DNA adaptor.

An expression vector for the protein of the present invention can be produced for example by (A) cutting the desired DNA fragment off from the DNA encoding for the protein of the present invention and then (B) ligating said DNA fragment to a region downstream from a promoter in a suitable expression vector.

The vector used includes E. coli-derived plasmid (e.g., pBR322, pBR325, pUC12, pUC13), Bacillus subtilis-derived plasmid (e.g., pUB110, pTP5, pC194), yeast-derived plasmid (e.g., pSH19, pSH15), bacteriophage such as λ-phage, and animal viruses such as retrovirus, vaccinia virus and Baculovirus, as well as pA1-11, pXT1, pRc/CMV, pRc/RSV, pcDNAI/Neo etc.

The promoter used in the present invention may be any suitable promoter compatible with a host used for expression of the gene. For example, when animal cells are used as the host, mention is made of SRα promoter, SV40 promoter, HIV-LTR promoter, CMV promoter, HSV-TK promoter etc.

Among these promoters, CMV (cytomegalovirus) promoter, SRa promoter etc. are preferably used. It is preferable to use trp promoter, lac promoter, recA promoter, λPL promoter, lpp promoter, T7 promoter etc. for microorganisms of the genus Escherichia as the host, SPO1 promoter, SPO2 promoter, penP promoter etc. for microorganisms of the genus Bacillus as the host, and PHO5 promoter, PGK promoter, GAP promoter, ADH promoter etc. for yeasts as the host. When insect cells are used as the host, polyhedron promoter, P10 promoter etc. are preferable.

The expression vector may contain an enhancer, a splicing signal, a poly A-added signal, a selective marker, an SV40 origin of replication (also referred to hereinafter as SV40 ori) etc. in addition to the promoter described above. The selective marker includes, for example, dihydrofolate reductase (also referred to hereinafter as dhfr) gene [methotrexate (MTX) resistance], ampicillin resistance gene (also referred to hereinafter as Amp^{r}) and neomycin resistance gene (G418 resistance, also referred to hereinafter as Neo^{r}). In particular, if the dhfr gene is used as a selective marker for dhfr gene-defective Chinese hamster cells, the desired gene can also be selected in a thymidine-free medium.

A signal sequence compatible with the host is added as necessary to a base sequence for the N-terminal of the protein of the present invention. A PhoA signal sequence, Omp A signal sequence etc. can be utilized for microorganisms of the genus Escherichia used as the host; an α-amylase signal sequence, subtilisin signal sequence etc. for microorganisms of the genus Bacillus as the host; an MFα signal sequence, SUC2 signal sequence etc. for yeasts as the host; and an insulin signal sequence, α-interferon signal sequence, antibody molecule signal sequence etc. for animal cells as the host.

The thus constructed vector containing the DNA encoding for the protein of the present invention can be used to produce transformants.

The microorganisms of the genus Escherichia, the microorganisms of the genus Bacillus, yeasts, insect cells, insects, animal cells etc. are used as the host.

The microorganisms of the genus Escherichia used include, for example, Escherichia coli K12 DH1 (Proc. Natl. Acad. Sci. USA, 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

The microorganisms of the genus Bacillus used include, for example, Bacillus subtilis MI114 (Gene, 24, 255 (1983)), 207-21 (Journal of Biochemistry, 95, 87 (1984)), etc.

The yeasts used include Saccharomyces cerevisiae AH22, AH22R⁻, NA87-11A, DKD-5D, 20B-12, Schizosaccharomyces pombe NCYC1913, NCYC2036, Pichia pastoris KM71, etc.

The insect cells used when the virus is AcNPV include, for example, cells (Spodoptera frugiperda cells; Sf cells) from an established cell line derived from caterpillars of Spodoptera frugiperda, MG1 cells derived from the midgut in Trichoplusia ni, High Five™ cells derived from eggs of Trichoplusia ni, cells derived from Mamestra brassicae and cells derived from Estigmena acrea. When the virus is BmNPV, cells (Bombyx mori N cells; BmN cells) from an established cell line derived from silkworms, etc., are used. The Sf cells used include, for example, Sf9 cells (ATCC CRL1711), Sf21 cells (Vaughn, J. L. et al., In Vivo, 13, 213-217 (1977)), etc.

The insects used include, for example, silkworm caterpillars (Maeda et al., Nature, 315, 592 (1985)).

The animals cells used include, for example, simian cell COS-7 (COS7), Vero, Chinese hamster ovary cells (abbreviated hereinafter to CHO cells), dhfr gene-defect Chinese hamster ovary cells (abbreviated hereinafter to CHO (dhfr⁻) cells), mouse L cells, mouse AtT-20, mouse myeloma cells, rat GH3, human FL cells etc.

The microorganisms of the genus Escherichia can be transformed according to a method described in e.g. Proc. Natl. Acad. Sci. USA, 69, 2110 (1972) or Gene, 17, 107 (1982).

The microorganisms of the genus Bacillus can be transformed according to amethod described in e. g. Molecular & General Genetics, 168, 111 (1979), etc.

The yeasts can be transformed according to amethod described in e.g. Methods in Enzymology, 194, 182-187 (1991), Proc. Natl. Acad. Sci. USA, 75, 1929 (1978), etc.

The insect cells or insects can be transformed according to a method described in e.g. Bio/Technology, 6, 47-55 (1988), etc.

The animal cells can be transformed according to a method described in e.g. "Saibo Kogaku Bessatsu 8, Shin-Saibo Kogaku Jikken Protocol (Cell Technology, Extra Number 8, New Experimental Protocol in Cell Technology)", 263-267 (1995) (published by Shujunsha), Virology, 52, 456 (1973), etc.

Transformants transformed with the expression vector containing DNA encoding for the protein can be thus obtained.

When transformants derived from the microorganisms of the genus Escherichia or Bacillus as the host are cultured, the medium used for their culture is preferably a liquid medium containing a carbon source, a nitrogen source, inorganic matter etc. necessary for growth of the transformants. The carbon source includes, for example, glucose, dextrin, soluble starch, sucrose etc.; the nitrogen source includes, for example, inorganic or organic materials such as ammonium salts, nitrates, corn steep liquor, peptone, casein, meat extract, soybean cake and potato extract; and the inorganic matter includes, for example, calcium chloride, sodium dihydrogen phosphate, magnesium chloride etc. In addition, yeast, vitamins, growth promoters etc. may be added. The pH value of the medium is desirably about 5 to 8.

For example, the medium for culturing the microorganisms of the genus Escherichia is preferably M9 medium containing glucose and casamino acid (Miller, Journal of Experiments in Molecular Genetics, 431-433, Cold Spring Harbor Laboratory, New York, 1972). To permit the promoter to work efficiently, a chemical such as 3β-indolylacrylic acid can be added as necessary.

The transformants from the microorganisms of the genus Escherichia as the host are cultured usually at about 15 to 43° C for about 3 to 24 hours during which the medium may be aerated or stirred as necessary.

The transformants from the microorganisms of the genus Bacillus as the host are cultured usually at about 30 to 40°C for about 6 to 24 hours during which the medium may be aerated or stirred as necessary.

The medium used for culturing the transformants from yeasts as the host includes, for example, Burkholder minimum medium (Bostian, K. L. et al., Proc. Natl. Acad. Sci. USA, 77, 4505 (1980)) and SD medium containing 0.5% casamino acid (Bitter, G. A. et al., Proc. Natl. Acad. Sci. USA, 81, 5330 (1984)). The pH value of the medium is adjusted preferably to about 5 to 8. The transformants are cultured usually at about 20 to 35°C for about 24 to 72 hours during which the medium may be aerated or stirred as necessary.

The medium used for culturing the transformants from insect cells or insects as the host includes, for example, a medium, if necessary prepared by adding inactivated additives such as 10% bovine serum to Grace's insect medium (Grace, T. C. C., Nature, 195, 788(1962)). The pH value of the medium is adjusted preferably to about 6.2 to 6.4. The transformants are cultured usually at about 27°C for about 3 to 5 days during which the medium may be aerated or stirred as necessary.

The medium used for culturing the transformants from animal cells as the host includes, for example, MEM medium containing about 5 to 20% fetal bovine serum (Science, 122, 501 (1952)), DMEMmedium (Virology, 8, 396 (1959)), RPMI 1640 medium (The Journal of the American Medical Association, 199, 519 (1967)), 199 medium (Proceeding of the Society for the Biological Medicine, 73, 1 (1950)) etc. The pH value is preferably about 6 to 8. The transformants are cultured usually at about 30 to 40 °C for about 15 to 60 hours during which the medium may be aerated or stirred as necessary.

The protein of the present invention can be formed on cytoplasmic membranes of the transformants.

From the resulting culture, the protein of the present invention can be separated and purified for example in the following manner.

To extract the protein of the present invention from the cultured microorganisms or cells, the cultured microorganisms or cells are collected in a usual manner, suspended in a suitable buffer, disrupted by sonication, lysozyme and/or freezing and thawing, and centrifuged or filtered to give a crude extract of the protein. The buffer may contain protein denaturants such as urea and guanidine hydrochloride and surfactants such as Triton X-100™. When the protein is secreted into the culture liquid, the culture supernatant is collected by separating the supernatant from the cultured microorganisms or cells by a per se known method.

The culture supernatant thus obtained, or the protein contained in the extract, can be purified by a suitable combination of separation and purification techniques known per se. These known separation and purification techniques make use of a method of utilizing solubility, such as salting-out and solvent precipitation, a method of mainly utilizing a difference in molecular weight, such as dialysis, ultrafiltration, gel filtration, and SDS-polyacrylamide gel electrophoresis, a method of utilizing a difference in electric charge, such as ion-exchange chromatography, a method of utilizing specific affinity, such as affinity chromatography, a method of utilizing a difference in hydrophobicity, such as reverse-phase HPLC, a method of utilizing a difference in isoelectric point, such as isoelectric focusing.

If the protein thus obtained is in a free form, it can be converted into a salt by a per se known method or its analogous method, while if the resulting protein is obtained in the form of a salt, it can be converted into a free protein or another salt by a per se known method or its analogous method.

Before or after purification, the protein produced by the transformants can be arbitrarily modified or its partial polypeptide can be removed by allowing a suitable protein-modifying enzyme to act on the protein. For example, trypsin, chymotrypsin, arginyl endopeptidase, protein kinase or glycosidase is used as the protein-modifying enzyme.

The thus formed protein of the present invention or a salt thereof can be measured by enzyme immunoassays or Western blotting with specific antibody.

The antibody against the protein and partial peptide of the present invention or a salt thereof may be a polyclonal or monoclonal antibody capable of recognizing the protein or partial peptide of the present invention or a salt thereof.

The antibody against the protein and partial peptide of the present invention or a salt thereof (referred to collectively as the protein of the present invention in the following description of the antibody) can be produced by a known process for producing antibody or antiserum by using the protein of the present invention as the antigen.

### [Preparation of a monoclonal antibody]

### (a) Preparation of monoclonal antibody-producing cells

The protein of the present invention is administered alone or together with a carrier or a diluent into warm-blooded animals at a site where the antibody can be produced by administration. To enhance the ability of the animals upon administration to produce the antibody, complete Freund's adjuvant or incomplete Freund's adjuvant may be administered. Administration is conducted usually once every 2 to 6 weeks and about 2 to 10 times in total. The warm-blooded animals used include, for example, monkey, rabbit, dog, guinea pig, mouse, rat, sheep, goat and chicken, among which mouse and rat are preferably used.

For production of the monoclonal antibody-producing cells, those animals having antibody titer are selected from the warm-blooded animals (e.g. mice) immunized with the antigen, and on the second to fifth day after the final immunization, their spleens or lymph nodes are collected, and the antibody-producing cells contained therein are fused with myeloma cells from animals of the same or different species, whereby monoclonal antibody-producing hybridomas can be produced. The antibody titer in antiserum can be measured for example by reacting the antiserum with the labeled protein described below and then measuring the activity of the labeling agent bound to the antibody. Fusion can be carried out by a known method such as the method of Keller and Millstein (Nature, 256, 495 (1975)). The fusion promoter includes polyethylene glycol (PEG) and Sendai virus, and PEG is preferably used.

The myeloma cells include myeloma cells from warm-blooded animals, such as NS-1, P3U1, SP2/0 and AP-1 among which P3U1 is preferably used. The ratio of the antibody-producing cells (spleen cells) to the myeloma cells used is from 1 : 1 to 20 : 1, and cell fusion can be effected efficiently by incubating the cells for about 1 to 10 minutes at 20 to 40 °C, preferably 30 to 37 °C, in the presence of PEG (preferably PEG 1000 to PEG 6000) at a concentration of about 10 to 80%.

The monoclonal antibody-producing hybridoma can be screened by various methods, for example by adding a culture supernatant of the hybridoma to a solid phase (e.g., a microplate) having the protein antibody adsorbed thereon directly or along with a carrier and then adding a radioactive substance- or enzyme-labeled anti-immunoglobulin antibody (which is e.g. an anti-mouse immunoglobulin antibody when mouse cells are subjected to cell fusion) or protein A to detect the monoclonal antibody bound to the solid phase or by adding a culture supernatant of the hybridoma to a solid phase having an anti-immunoglobulin antibody or protein A adsorbed thereon and then adding the protein labeled with a radioactive substance or an enzyme to detect the monoclonal antibody bound to the solid phase.

The monoclonal antibody can be screened according to a per se known method or its analogous manner. Screening can be carried out usually in an animal cell culture medium to which HAT (hypoxanthine, aminopterin, thymidine) was added. The screening and breeding medium may be any medium in which the hybridoma can grow. Examples of such medium include PRMI 1640 medium containing 1 to 20% (preferably 10 to 20%) FBS, GIT medium containing 1 to 10% FBS (Wako Pure Chemical Industries, Ltd.), and a serum-free medium for hybridoma culture (SFM-101, Nissui Pharmaceutical, Co., Ltd.). The culture temperature is usually 20 to 40 °C, preferably about 37 °C. The culture time is usually 5 days to 3 weeks, preferably 1 to 2 weeks. Culture can be conducted usually in 5% CO₂ gas. The antibody titer in a culture supernatant of the hybridoma can be measured in the same manner as in the measurement of the antibody titer in antiserum as described above.

### (b) Purification of the monoclonal antibody

The monoclonal antibody can be separated and purified by per se known techniques, for example by techniques of separating and purifying immunoglobulin, such as salting-out, alcohol precipitation, isoelectric precipitation, electrophoresis, absorption-desorption with an ion-exchanger (e.g. DEAE), ultracentrifugation and gel filtration or by specific purification of the antibody by collecting it with an active adsorbent such as antigen-bound solid phase, protein A or protein G and then eluting the antibody therefrom.

### [Preparation of a polyclonal antibody]

The polyclonal antibody of the present invention can be produced by a per se known method or its analogous method. For example, the desired polyclonal antibody can be produced by preparing the immune antigen (protein antigen) or a conjugate thereof with a carrier protein, then immunizing warm-blooded animals therewith in the same manner as in production of the monoclonal antibody as described above, collecting a material containing the antibody against the protein of the present invention from the immunized animals, and separating and purifying the antibody.

For the conjugate of the immune antigen with a carrier protein used for immunizing warm-blooded animals, the type of the carrier protein and the mixing ratio of the carrier to the hapten are not particularly limited insofar as the desired antibody can be efficiently produced by immunization with the antigen crosslinked via the hapten with the carrier, and for example, the conjugate is produced by coupling the hapten with bovine serum albumin, bovine cyloglobulin, hemocyanin or the like in a ratio of 1 to about 0.1 to 20, preferably about 1 to 5.

The hapten can be coupled with the carrier by use of various condensing agents such as glutaraldehyde, carbodiimide, maleimide-activated ester, and activated ester reagents containing thiol group and dithiopyridyl group.

The resulting condensation product is administered alone or together with a carrier or a diluent into warm-blooded animals at a site where the antibody can be produced. To enhance the ability of the animals upon administration to produce the antibody, complete Freund's adjuvant or incomplete Freund's adjuvant may be administered. Administration is conducted usually once every 2 to 6 weeks and about 3 to 10 times in total.

The polyclonal antibody can be collected form blood, ascites etc. preferably blood in the warm-blooded animals immunized by the method described above.

The polyclonal antibody titer in antiserum can be measured in the same manner as in the measurement of the antibody titer in antiserum as described above. Separation and purification of the polyclonal antibody can be carried out by a method of separating and purifying immunoglobulin, which is similar to the separation and purification of the monoclonal antibody as described above.

Antisense DNA having a base sequence complementary or substantially complementary to the DNA (referred to as the DNA of the present invention in the following description of antisense DNA) encoding for the protein or partial peptide of the present invention may be any antisense DNA having a base sequence complementary or substantially complementary to the DNA of the present invention and having an ability to suppress expression of said DNA.

The base sequence substantially complementary to the DNA of the present invention includes, for example, base sequences having about 70% or more, preferably about 80% or more, more preferably about 90% or more, and most preferably about 95% or more homology to the whole or a part of a base sequence (that is, a complementary chain of the DNA of the present invention) complementary to the DNA of the present invention. Particularly preferred is an antisense DNA having about 70% or more, preferably about 80% or more, more preferably about 90% or more, and most preferably about 95% or more homology to that partial base sequence which in the base sequence of the complementary chain of the DNA of the present invention, encodes for an N-terminal region of the protein of the present invention (e.g., a base sequence in the vicinity of the initiation codon). Such antisense DNA can be produced, for example, by using a known DNA synthesizer.

The protein of the present invention usually has a signal peptide and is thus efficiently secreted extracellularly, and has an important biological activity for signal transfer and self-protection as a humoral factor.

Use of the protein or partial peptide of the present invention or a salt thereof (also referred to hereinafter as the protein etc. of the present invention), the DNA encoding for the protein or partial peptide of the present invention (also referred to hereinafter as the DNA of the present invention), the antibody against the protein or partial peptide of the present invention or against salts thereof (also referred to hereinafter as the antibody of the present invention), and the antisense DNA is described as follows.
(1) Because the protein of the present invention is tissue-specifically expressed, it can be used as a tissue marker. That is, the protein of the present invention is useful as a marker for detecting tissue differentiation, morbid state, cancer metastasis etc. Further, it can be used for separation of its corresponding receptor, ligand, binding protein etc. Further, it can be utilized in a panel for high through-put screening known per se in order to examine biological activity. It can also be used for study of hereditary diseases by chromosome mapping.
(2) Treating or preventing agent for various diseases that the protein of the present invention is associated with
   The protein etc. of the present invention occur as a humoral factor in a living body so that when the protein etc. of the present invention or the DNA of the present invention is abnormal or defective or its expression is abnormally reduced or promoted, there occur various diseases such as immune diseases, pulmonary insufficiency, pancreatic insufficiency, infectious diseases or gastrointestinal disorders.
   Accordingly, the protein etc. of the present invention and the DNA of the present invention can be used as pharmaceutical preparations such as treating or preventing agents for immune diseases, pulmonary insufficiency, pancreatic insufficiency, infectious diseases or gastrointestinal disorders.
   More specifically, the protein etc. of the present invention and the DNA of the present invention can be used as, for example, pharmaceutical preparations such as treating or preventing agents for diseases such as trachea- and bronchus-related diseases (e.g., bronchitis, influenza infectious diseases, bronchial asthma, upper trachea inflammation, bronchiectasis etc.), lung-related diseases (lung cancer, tuberculosis, pneumonia, pulmonary emphysema, pulmonary infarct, pulmonary stagnation, respiratory insufficiency, cystic pulmonary fibroma, pulmonary sarcoidosis, pulmonary edema, pulmonary high blood pressure, pneumoconiosis etc.), stomach-related diseases (e.g., *Helicobacter pylori* infectious diseases, digestive ulcer, gastric cancer, stomach atony, gastritis, Mallory-Weiss syndrome, stomach expansion, abnormal gastric juice secretion, Menetrier's disease (gigantic hypertrophic gastritis), stomach tetany, torso disease (stomach dizziness), gastrointestinal neurosis, etc.), diabetes, digestive insufficiency, maintenance of enteric bacteria, etc.
   For example, those patients who cannot sufficiently or normally exhibit information transfer in cells because of a reduction or defect in the protein etc. of the present invention in the living body can be treated for sufficiently or normally exhibiting the role of the protein etc. of the present invention by (A) administering the DNA of the present invention into the patients and expressing the protein etc. of the present invention in the living body, (B) inserting the DNA of the present invention into cells to express the protein etc. of the present invention and then transplanting said cells in the patients, or (C) by administering the protein etc. of the present invention into the patients.
   To use the DNA of the present invention as the treating or preventing agent described above, said DNA can be administered in a usual manner into humans or warm-blooded animals directly or after insertion thereof into a suitable vector such as retrovirus vector, adenovirus vector and adenovirus-associated virus vector. The DNA of the present invention is used alone or along with physiologically acceptable carriers such as an auxiliary agent for promoting ingestion, to form a pharmaceutical preparation which can be administered via a gene gun or a catheter such as hydrogel catheter.
   The protein etc. of the present invention used as the treating or preventing agent described above is preferably the one having a purity of at least 90%, preferably 95% or more, more preferably 98% or more, and most preferably 99% or more.
   For example, the protein etc. of the present invention can be used orally as tablets coated with sugar as necessary, capsules, elixirs and microcapsules or parenterally as an aseptic solution with water or with other pharmaceutically acceptable solutions or as an injection such as suspension. For example, these preparations can be produced by admixing physiologically acceptable carriers, flavors, excipients, vehicles, preservatives, stabilizers and binders with the protein etc. of the present invention in a unit dose required for generally approved pharmaceutical manufacturing. The amount of the active ingredient in these pharmaceutical preparations is designed to have a suitable capacity in the designated range.
   The additives which can be admixed with the tablets, capsules etc. include, for example, binders such as gelatin, corn starch, tragacanth, gum arabic, excipients such as crystalline cellulose, swelling agents such as corn starch, gelatin and alginic acid, lubricants such as magnesium stearate, sweeteners such as sucrose, lactose and saccharine, and flavors such as peppermint, akamono oil and cherry. When one capsule is in a unit form, liquid carriers such as fats and oils can be contained in the materials described above. The aseptic composition for injection can be formulated according to conventional pharmaceutical manufacturing by dissolving or suspending the active material and naturally occurring vegetable oils such as sesame oil and coconut oil in vehicles such as injection water.
   The aqueous solution for injection includes, for example, physiological saline or an isotonic solution containing glucose and other supplementary agents (e.g., D-sorbitol, D-mannitol, sodium chloride etc.), and may be used in combination with suitable solubilizer such as alcohols (e.g., ethanol etc.), polyalcohols (e.g., propylene glycol, polyethylene glycol etc.) and nonionic surfactants (e.g., Polysorbate 80™, HCO-50 etc.). The oily solution includes, for example, sesame oil, soybean oil etc., and may be used in combination with solubilizer such as benzyl benzoate, benzyl alcohol etc. Further, it may be compounded with the buffer (e.g., phosphate buffer, sodium acetate buffer etc.), soothing agents (e.g., benzalkonium chloride, procaine hydrochloride etc.), stabilizers (e.g., human serum albumin, polyethylene glycol etc.), preservatives (e.g., benzyl alcohol, phenol etc.), antioxidants etc. Usually, the prepared injection is introduced into suitable ampoules.
   The vector having the DNA of the present invention inserted therein can also be formed into a pharmaceutical preparation in the same manner as described above and usually parenterally administered.
   The pharmaceutical preparation thus obtained is safe and low toxic so that it can be administered into e.g. humans or warm-blooded animals (e.g., rat, mouse, guinea pig, rabbit, bird, sheep, pig, cow, horse, cat, dog, monkey and chimpanzee).
   The dose of the protein etc. of the present invention is varied depending on the intended diseases, the subject of administration, administration route etc., and when the protein etc. of the present invention are orally administered for the purpose of treatment of immune diseases, the protein etc. are administered usually in a dose of about 1 to 1000 mg, preferably about 10 to 500 mg and more preferably about 10 to 200 mg every day into a (60 kg) adult. For parenteral administration, the dose of the protein etc. of the present invention is varied depending on the subject of administration, the intended diseases etc., and when the protein etc. of the present invention are administered in the form of an injection for the purpose of treatment of immune diseases, the protein etc. are conveniently injected into an affected part of a (60 kg) adult in a daily dose of about 1 to 1000mg, preferably about 1 to 200 mg and more preferably about 10 to 100 mg. The protein etc. of the present invention can also be administered into other animals in the same dose (per 60 kg) as described above.
(3) Screening of candidates for pharmaceutical compounds for treatment of diseases
   The protein etc. of the present invention occur as a humoral factor in a living body (particularly in the lungs, trachea, stomach, pancreas etc.), and thus compound or a salt thereof which promotes the functions of the protein etc. of the present invention can be used as pharmaceutical preparations such as treating or preventing agents for immune diseases, pulmonary insufficiency, pancreatic insufficiency, infectious diseases or gastrointestinal disorders.
   On the other hand, a compound or a salt thereof which inhibits the functions of the protein etc. of the present invention can be used as pharmaceutical preparations such as treating or preventing agents for diseases attributable to excessive production of the protein etc. of the present invention.
   Accordingly, the protein etc. of the present invention are useful as reagents for screening for a compound or a salt thereof which promotes or inhibits the functions of the protein etc. of the present invention.
   That is, the present invention provides a method of screening for i) a compound or a salt thereof (sometimes referred to hereinafter as promoter) promoting the functions of the protein or partial peptide of the present invention or a salt thereof or ii) a compound (sometimes referred to hereinafter as inhibitor) inhibiting the functions of the protein or partial peptide of the present invention or a salt thereof, which comprises use of the protein or partial peptide of the present invention or a salt thereof.
   The kit for screening of the present invention comprises the protein or partial peptide of the present invention or a salt thereof.
   The compound or a salt thereof which can be obtained by use of the method of screening or the kit for screening of the present invention is a compound selected from, for example, peptides, proteins, non-peptide compounds, synthetic compounds, fermentation products, cellular extracts, plant extracts, animal tissue extracts and plasma, and promotes or inhibits the functions of the protein etc. of the present invention.
   As the salts of said compound, the same salts as those of the protein of the present invention are used.
   If the compound obtained by the method of screening or kit for screening of the present invention is used as the treating or preventing agent described above, said compound can be formed in a usual manner into a pharmaceutical preparation. For example, said compound can be formed into tablets, capsules, elixirs, microcapsules, aseptic solutions and suspensions in the same manner as in production of the pharmaceutical preparation containing the protein etc. of the present invention as described above.
   The pharmaceutical preparation thus obtained is safe and low toxic so that it can be administered into e.g. humans or warm-blooded animals (e.g., mouse, rat, rabbit, sheep, pig, cow, horse, bird, cat, dog, monkey, chimpanzee etc.).
   The dose of said compound or a salt thereof is varied depending on its action, the intended diseases, the subject of administration, administration route etc., and when the compound promoting the functions of the protein etc. of the present invention is orally administered for the purpose of treatment of inflammatory diseases, said compound is administered in a dose of usually about 0.1 to 100 mg, preferably about 1.0 to 50 mg and more preferably about 1.0 to 20 mg every day into a (60 kg) adult. For parenteral administration, the dose of said compound is varied depending on the subject of administration, the intended diseases etc., and when the compound promoting the functions of the protein etc. of the present invention is administered in the form of an injection for the purpose of treatment of inflammatory diseases, said compound is conveniently injected intravenously in a dose of usually about 0.01 to 30 mg, preferably about 0.1 to 20 mg and more preferably about 0.1 to 10 mg every day into a (60 kg) adult. Said compound can be administered into other animals in the same dose (per 60 kg) as described above.
   On the other hand, when the compound inhibiting the functions of the protein etc. of the present invention is orally administered, said compound is administered in a dose of usually about 0.1 to 100 mg, preferably about 1.0 to 50 mg and more preferably about 1.0 to 20 mg every day into a (60 kg) adult. For parenteral administration, the dose of said compound is varied depending on the subject of administration, the intended diseases etc., and when the compound inhibiting the functions of the protein etc. of the present invention is administered in the form of an injection, said compound is conveniently injected intravenously in a dose of usually about 0.01 to 30 mg, preferably about 0.1 to 20 mg and more preferably about 0.1 to 10 mg every day into a (60 kg) adult. Said compound can be administered into other animals in the same dose (per 60 kg) as described above.
(3) Quantification of the protein or partial peptide of the present invention or a salt thereof
   The antibody against the protein etc. of the present invention (sometimes referred to hereinafter as the antibody of the present invention) can specifically recognize the protein etc. of the present invention so that it can be used for e.g. quantification of the protein etc. of the present invention in a test sample, particularly for quantification thereof by sandwich immunoassays.
   That is, the present invention provides:
   (i) A method of quantifying the protein etc. of the present invention in a test solution, which comprises allowing a test solution and the labeled protein etc. of the present invention to react competitively with the antibody of the present invention and determining the ratio of the labeled protein etc. of the present invention bound to said antibody; and
   (ii) A method of quantifying the protein etc. of the present invention in a test solution, which comprises allowing a test solution to react with the antibody of the present invention insolubilized on a carrier and another labeled antibody of the present invention simultaneously or successively and then measuring the activity of the labeling agent on the insolubilized carrier.

   The monoclonal antibody against the protein etc. of the present invention (hereinafter, also referred to as the monoclonal antibody of the present invention) can be used not only for quantifying the protein etc. of the present invention but also for detection thereof by tissue staining etc. For these purposes, the antibody molecule itself or an F(ab')₂, Fab' or Fab fraction of the antibody molecule may be used.
   The method of quantifying the protein etc. of the present invention by using the antibody of the present invention is not particularly limited insofar as the method comprises detecting the amount of the antibody, the antigen or an antigen-antibody conjugate, corresponding to the amount of the antigen (e.g., the amount of the protein) in a test solution, by chemical or physical means and calculating it on the basis of a standard curve prepared using standard solutions containing known amounts of the antigen. For example, nephelometry, the competitive method, immunometric method and sandwich method are preferably used, but the sandwich method described below is particularly preferably used for sensitivity and specificity.
   The labeling agent used in the measurement method using a labeling substance includes, for example, a radioisotope, enzyme, fluorescent material and luminescent material. The radioisotope includes, for example, [¹²⁵I], [¹³¹I], [³H], [¹⁴C], and so forth. The enzyme described above is preferably a stable enzyme with high specific activity, which includes, for example, β - galactosidase, β-glucosidase, alkali phosphatase, peroxidase and malate dehydrogenase. The fluorescent material includes, for example, fluorescamine and fluorescein isocyanate. The luminescent material includes, for example, luminol, luminol derivatives, luciferin and lucigenin. Further, a biotin-avidin system can also be used for binding the labeling agent to the antibody or antigen.
   The antigen or antibody may be insolubilized by physical adsorption or via chemical bonding used conventionally for insolubilization or immobilization of proteins, enzymes etc. The carrier includes insoluble polysaccharides such as agarose, dextran and cellulose, synthetic resin such as polystyrene, polyacrylamide and silicon, and glass etc.
   In the sandwich method, a test sample is allowed to react with the insolubilized monoclonal antibody of the present invention (primary reaction), then another insolubilized monoclonal antibody of the present invention is allowed to react therewith (secondary reaction), and the activity of the labeling agent on the insolubilized carrier is measured, whereby the protein of the present invention in the test sample can be quantitatively determined. The primary and secondary reactions may be carried out in the reverse order, simultaneously, or separately after a predetermined time. The labeling agent and the method of insolubilization may be in accordance with those described above. For the purpose of improving measurement sensitivity etc., immunoassays by the sandwich method may make use of not only one kind of antibody but also a mixture of two or more kinds of antibody as the solid-phase antibody or labeling antibody.
   In the method of measuring the protein etc. of the present invention by the sandwich method according to the present invention, the monoclonal antibodies of the present invention used in the primary and secondary reactions are preferably those antibodies to which the protein etc. of the present invention are bound at different sites. That is, when the antibody used in e.g. the secondary reaction recognizes the C-terminal region of the protein etc. of the present invention, the other antibody used in the primary reaction recognizes other region (e.g. the N-terminal region) than the C-terminal region.
   The monoclonal antibody of the present invention can be used not only in the sandwich method but also in other measurement systems such as competitive method, immunometric method and nephelometry.
   In the competitive method, an antigen in a test sample and its labeled antigen are allowed to react competitively with the antibody, then the unreacted labeled antigen (F) and the labeled antibody (B) bound to the antibody are separated from each other (B/F separation), and the amount of either labeled B or F is determined to quantify the amount of the antigen in the sample. This reaction method employs either a liquid-phase method in which a soluble antibody is used as the antibody and polyethylene glycol and a second antibody against the above antibody are used in B/F separation, or a solid-phase method in which a solid-phase (or immobilized) antibody is used as the first antibody, or a soluble antibody is used as the first antibody while a solid-phase antibody is used as the second antibody.
   In the immunometric method, the antigen in a test solution and the solid-phase (or immobilized) antigen are allowed to react competitively with the labeled antibody present in a predetermined amount, followed by separating the solid phase from the liquid phase, or alternatively the antigen in a test solution is allowed to react with the labeled antibody present in excess, and then the solid-phase antigen is added thereto to permit the unreacted labeled antibody to be bound to the solid phase, followed by separating the solid phase from the liquid phase. Then, the amount of the label in either phase is measured to quantify the amount of the antigen in the test solution.
   In nephelometry, the amount of insoluble precipitates in gel or solution, formed by antigen-antibody reaction, is measured. Laser nephelometry using laser scattering can be applied preferably to the case where precipitates are obtained in a small amount because of a very small amount of antigen in a test solution.
   For application of these immunoassays to the quantification method of the present invention, it is not necessary to establish special conditions, procedures etc. A measurement system for the protein etc. of the present invention can be established in an ordinary manner by those skilled in the art by modifying the conventional conditions and procedures. The details of these general technical means can be referred to in general remarks, books etc.
   For example, reference can be made of "Radioimmunoassays" edited by Hirhoshi Irie (published by Kodansha in 1974), "Radioimmunoassays" (2nd edition) edited by Hiroshi Irie (published by Kodansha in 1979), "Enzyme Immunoassays" edited by Eiji Ishikawa (published by Igakushoin in 1978), "Enzyme Immunoassays" (2nd edition) edited by Eiji Ishikawa (published by Igakushoin in 1982), "Enzyme Immunoassays" (3rd edition) edited by Eiji Ishikawa (published by Igakushoin in 1987), Methods in Enzymology, Vol. 70 (Immunochemical Techniques (Part A), Methods in Enzymology, Vol. 73 (Immunochemical Techniques (Part B)), Methods in Enzymology, Vol. 74 (Immunochemical Techniques (Part C)), Methods in Enzymology, Vol. 84 (Immunochemical Techniques (Part D: Selected Immunoassays)), and Methods in Enzymology, Vol. 92 (Immunochemical Techniques (Part E: Monoclonal Antibodies and General Immunoassay Methods)), Methods in Enzymology, Vol. 121 (Immunochemical Techniques (Part I: Hybridoma Technology and Monoclonal Antibodies)) (which are published by Academic Press Ltd.).
   By using the antibody of the present invention in the manner as described above, the protein etc. of the present invention can be quantified with good sensitivity.
   If a reduction in the protein etc. of the present invention is detected by quantifying the concentration of the protein etc. of the present invention by the antibody of the present invention, the examinee can be diagnosed as having diseases such as immune diseases, pulmonary insufficiency, pancreatic insufficiency, infectious diseases or gastrointestinal disorders, or it is predicted that he will probably have such diseases.
   Further, the antibody of the present invention can be used to detect the protein etc. of the present invention present in humor and tissues. The antibody of the present invention can also be used for preparation of an antibody column used in purification of the protein etc. of the present invention, for detection of the protein etc. of the present invention in each fraction during purification, and for analysis of the behavior of the protein etc. of the present invention in cells to be examined.
(5) Genetic diagnostic agent
   Because the DNA of the present invention can be used as, for example, a probe to detect abnormalities (genetic abnormalities) in DNA or mRNA encoding for the protein or partial peptide of the present invention in humans or warm-blooded animals (e.g., rat, mouse, guinea pig, rabbit, bird, sheep, pig, cow, horse, cat, dog, monkey, chimpanzee etc.), the DNA of the present invention is useful as a genetic diagnostic agent for e.g. impairment, mutation or depressed expression of said DNA or mRNA or an increase or excessive expression of said DNA or mRNA.
   This genetic diagnosis using the DNA of the present invention can be carried out by the per se known techniques, for example Northern hybridization and the PCR-SSCP method (Genomics, Vol. 5, pp. 874-879 (1989) and Proceedings of the National Academy of Sciences of the United States of America, Vol. 86, pp. 2766-2770 (1989)).
   For example, when the depressed expression is detected by Northern hybridization or a mutation in the DNA is detected by the PCR-SSCP method, the examinee can be diagnosed as having diseases such as immune diseases, pulmonary insufficiency, pancreatic insufficiency, infectious diseases or gastrointestinal disorders.
(6) A pharmaceutical preparation comprising an antisense DNA
   Because the antisense DNA capable of binding complimentarily to the DNA of the present invention to suppress expression of said DNA can suppress the functions of the protein etc. of the present invention or the DNA of the present invention in a living body, the antisense DNA can be used as, for example, a treating or preventing agent for diseases attributable to excessive expression of the protein etc. of the present invention.
   When the antisense DNA is used as the treating or preventing agent, it can be brought into practice in the same manner as for the above-described agent containing the DNA of the present invention for treating or preventing various diseases.
   When the antisense DNA is used, said antisense DNA can be brought into practice in a usual manner directly or after insertion thereof into a suitable vector such as retrovirus vector, adenovirus vector and adenovirus-associated virus vector. The antisense DNA is used alone or along with physiologically acceptable carriers such as an auxiliary agent for promoting ingestion, to form a pharmaceutical preparation which can be administered via a gene gun or a catheter such as hydrogel catheter.
   Further, said antisense DNA can be used as a diagnostic oligonucleotide probe for examining the presence or expression of the DNA of the present invention in tissues or cells.
(7) A pharmaceutical preparation comprising an antibody of the present invention
   The antibody of the present invention having the action of neutralizing the activity of the protein etc. of the present invention can be used as a pharmaceutical preparation such as a treating or preventing agent for diseases attributable to excessive expression of the protein etc. of the present invention.
   The treating or preventing agent comprising an antibody of the present invention against the diseases described above can be administered orally or parenterally as a liquid agent directly or as a pharmaceutical composition in a suitable form into humans or mammals (e.g., rat, rabbit, sheep, pig, cow, cat, dog, monkey etc.). The dose is varied depending on the subject of administration, intended diseases, conditions, administration route etc., and the antibody of the present invention is conveniently injected intravenously once to about 5 times every day, preferably once to about 3 times in a dose of usually about 0.01 to 20 mg/kg, preferably about 0.1 to 10 mg/kg, and more preferably 0.1 to 5 mg/kg for each administration. A similar dose can be administered in other parenteral administration or oral administration. For particularly severe conditions, the dose may be increased depending on the conditions.
   The antibody of the present invention can be administered as such or as a suitable pharmaceutical composition. The pharmaceutical composition used in the administration described above contains the above antibody or a salt thereof, pharmaceutically acceptable carriers, diluent or excipients. The composition is provided in a pharmaceutical form suitable for oral or parenteral administration.
   That is, the composition for e.g. oral administration is in a solid or liquid form, specifically in the form of tablets (including sugar-coated tablets and film-coated tablets), pills, granules, powder, capsules (including soft capsules), syrups, emulsions, suspensions etc. The composition is produced in a per se known method and contains carriers, diluent or excipients generally used in the field of pharmaceutical manufacturing. For example, the carriers and excipients used in tablets include lactose, starch, sucrose, magnesium stearate etc.
   The composition used for parenteral administration includes, for example, injections, suppositories etc., and the injections are in pharmaceutical'forms such as intravenous injection, subcutaneous injection, intracutaneous injection, intramuscular injection and intravenous infusion. These injections are prepared in a usual manner by dissolving, suspending or emulsifying the antibody of the present invention or its salt in a germ-free, aqueous or oily solution used conventionally in injections. The aqueous solution for injection includes, for example, physiological saline or an isotonic solution containing glucose and other supplementary agents, and may be used in combination with suitable solubilizer such as alcohols (e.g., ethanol), polyalcohols (e.g., propylene glycol, polyethylene glycol) and nonionic surfactants [e.g., Polysorbate 80 and HCO-50 (polyoxyethylene (50 moles) adduct of hydrogenated castor oil)]. The oily solution includes, for example, sesame oil, soybean oil etc., and may be used in combination with solubilizer such as benzyl benzoate, benzyl alcohol etc. The prepared injection is introduced usually into suitable ampoules. A suppository used in administration into rectum is prepared by mixing the above antibody or its salt with a conventional base for suppositories.
   The oral or parenteral pharmaceutical preparation described above is produced conveniently in a unit form adaptable to the dose of the active ingredient. The pharmaceutical preparation in such a unit form includes, for example, tablets, pills, capsules, injections (ampoules) and suppositories, where the above antibody is contained in an amount of usually 5 to 500 mg/unit for these pharmaceutical forms and preferably 5 to 100 mg/unit for the injections or 10 to 250 mg/unit for the other pharmaceutical forms.
   The respective compositions described above may contain any other active ingredients that do not generate undesired interaction upon incorporation into the above antibody.
(8) DNA-transferred animals
   The present invention provides non-human mammals having the extraneous DNA encoding for the protein etc. of the present invention (sometimes referred to hereinafter as the extraneous DNA of the present invention) or its mutated DNA (sometimes referred to hereinafter as the extraneous mutated DNA of the present invention).
   That is, the present invention provides:
   [1] A non-human mammal having the extraneous DNA of the present invention or its mutated DNA;
   [2] The animal according to the above [1], wherein the non-human mammal is a rodent;
   [3] The animal according to the above [2], wherein the rodent is a mouse or rat; and
   [4] A recombinant vector which comprises the extraneous DNA of the present invention or its mutated DNA and be capable of expression in mammals.

   The non-human mammal having the extraneous DNA of the present invention or its mutated DNA (hereinafter, referred to as the DNA-transferred animals of the present invention) can be produced by transferring the intended DNA to embryonic cells such as unfertilized ova, fertilized ova, sperms and their initial cells, preferably at the stage of embryonic development in the development of non-human mammals (more preferably at the stage of single cells or fertilized ova and generally before the 8-cell stage) by the calcium phosphate method, electric pulse method, lipofection method, aggregation method, microinjection method, particle gun method or DEAE-dextran method. By the method of transferring the DNA, the intended extraneous DNA of the present invention can be transferred to somatic cells, organs in a living body, tissues and cells and utilized in cell culture and tissue culture, and these cells can also be fused with embryonic cells by the cell fusion per se known method in order to produce the DNA-transferred animals of the present invention.
   The non-human mammals used include, for example, cow, pig, sheep, goat, rabbit, dog, cat, guinea pig, hamster, mouse and rat. In particular, rodents, particularly mice (e.g., C57BL/6 line and DBA2 line as pure line and B6C3F₁ line, BDF₁ line, B6D2F₁ line, BALB/c line and ICR line as crossed line) and rats (e.g., Wistar, SD etc.), are preferable in formation of a morbid animal model system because of their easy breeding with a relatively short period of development and biological cycle.
   With respect to the recombinant vector capable of expression in mammals, the "mammals" include humans in addition to the non-human mammals described above.
   The extraneous DNA of the present invention is not the DNA of the present invention inherent in non-human mammals but the DNA of the present invention once isolated and extracted from mammals.
   The mutated DNA of the present invention includes DNA derived from the original DNA of the present invention by a mutation in the base sequence thereof, specifically by addition or deletion of some bases or by substitution of some bases by other bases, and the mutated DNA also includes abnormal DNA.
   The abnormal DNA refers to DNA expressing the abnormal protein of the present invention, and for example, DNA expressing a protein suppressing the functions of the normal protein of the present invention is used.
   The extraneous DNA of the present invention may be derived from animals of either the same species as or a different species from that of the intended animals. For transferring the DNA of the present invention to the intended animals, it is generally advantageous to use a DNA construct having the DNA bound to a region downstream from a promoter in order to express the DNA in the animal cells. To transfer e.g. the human DNA of the present invention, a DNA construct (e.g. a vector) having the human DNA of the present invention bound to a region downstream from a promoter permitting expression of DNA having high homology to the DNA of the present invention and derived from various mammals (e.g., rabbit, dog, cat, guinea pig, hamster, rat, mouse etc.) carrying the DNA of the present invention can be microinjected into fertilized ova of the intended animals, for example, mouse fertilized ova in order to produce DNA-transferred animals highly expressing the DNA of the present invention.
   The expression vector used for the protein of the present invention includes plasmids derived from Escherichia coli, Bacillus subtilis or yeast, bacteriophage such as λ-phage, retrovirus such as Moloney's leukemia virus, and animal viruses such as vaccinia virus and Baculovirus. In particular, the plasmids derived from Escherichia coli, Bacillus subtilis or yeast are preferably used.
   The promoter for regulating expression of the DNA includes (1) promoters in DNA derived from viruses (e.g., simian virus, cytomegalovirus, Moloney's leukemia virus, JC virus, breast cancer virus, poliovirus etc.), (2) promoters derived from various mammals (human, rabbit, dog, cat, guinea pig, hamster, rat, mouse etc.), for example, promoters for albumin, insulin II, uroplakin II, elastase, erythropoietin, endoserine, muscular creatine kinase, glia fibrous acidic protein, glutathione S-transferase, platelet-derived growth factor β, keratin K1, K10 and K14, collagen I and II type, cyclic AMP-dependent kinase βI subunit, dystrophin, tartaric acid-resistant alkali phosphatase, atrial natriuretic factor, endothelial receptor tyrosine kinase (abbreviated generally to Tie 2), sodium potassium adenosine triphosphatase (Na, K-ATPase), neurofilament light chain, metallothionein I and IIA, metalloproteinase 1 tissue inhibitor, MHC class I antigen (H-2L), H-ras, renin, dopamine β-hydroxylase, thyroid peroxidase (TPO), polypeptide chain-extending factor 1α (EF-1α), β-actin, α- and β-myosin heavy chains, myosin light chains 1 and 2, myelin basic protein, thyroglobulin, Thy-1, immunoglobulin, H chain variable region (VNP), serum amyloid P component, myoglobin, troponin, smooth muscular α-actin, preproenkephalin A and vasopressin. Among these promoters, promoters such as cytomegalovirus promoter, human polypeptide-extending factor 1α (EF-1α) promoter, human and chicken β actin promoters are preferable because of their ability to express the DNA in the whole body.
   Preferably, the vector has a sequence (generally called terminator) for terminating translation of the intended messenger RNA in the DNA-transferred mammals, and for example the sequence of each DNA derived from viruses or mammals, preferably SV40 terminator from simian virus, can be used.
   Further, a splicing signal for each DNA, an enhancer region, a part of introns from eukaryotic DNA, etc. can also be linked depending on the object to a 5' -upstream region from the promoter, to a region between the promoter and the translational region, or to a 3'-downstream region from the translational region.
   The translational region can be constructed as the DNA construct capable of expression in the DNA-transferred animals by conventional DNA engineering means for linking it to a downstream region from the promoter or if desired to an upstream region from the translational termination site.
   The extraneous DNA of the present invention is transferred to cells at the stage of fertilized egg cells so as to secure the presence of the DNA in all embryonic cells and somatic cells of the intended mammals. The presence of the extraneous DNA of the present invention in the embryonic cells in the produced animals after transfer of the DNA means that the extraneous DNA of the present invention is maintained in all embryonic cells and somatic cells in the offspring of the produced animals. The offspring of the produced animals has inherited the extraneous DNA of the present invention so that the extraneous DNA of the present invention is present in all embryonic cells and somatic cells of the offspring.
   After it is confirmed that the non-human mammals having the extraneous normal DNA of the present invention transferred to them maintain the extraneous DNA stably through crossbreeding, they can be bred generation after generation as animals carrying said DNA under normal breeding environments.
   The extraneous DNA of the present invention is transferred to cells at the stage of fertilized egg cells so as to secure the presence of the DNA in excess in all embryonic cells and somatic cells in the produced animals. The presence of the extraneous DNA of the present invention in excess in the embryonic cells in the produced animals after transfer of the DNA means that the extraneous DNA of the present invention is maintained in all embryonic cells and somatic cells in the offspring of the produced animals. The offspring of the produced animals has inherited the extraneous DNA of the present invention so that the extraneous DNA of the present invention occurs in excess in all embryonic cells and somatic cells of the offspring.
   Homozygote animals having the introduced DNA in both homogenous chromosomes are obtained, and these male and female animals are crossbred to produce the offspring having said DNA in excess and capable of breeding generation after generation.
   The non-human mammals having the normal DNA of the present invention can be used as a morbid animal model because the normal DNA of the present invention is highly expressed in these animals to promote the functions of endogenous normal DNA, resulting in the onset of functional exasperation by the protein of the present invention. For example, the normal DNA-transferred animals of the present invention can be used to elucidate the mechanism of the morbid functional exasperation caused by the protein of the present invention or those diseases connected with the protein of the present invention or to examine the method of treating these diseases.
   Further, the mammals having the extraneous normal DNA of the present invention transferred to them can also be used in a test for screening a treating agent against those diseases connected with the protein of the present invention because these animals have the symptom of an increase in the free protein of the present invention.
   On the other hand, the non-human mammals having the extraneous abnormal DNA of the present invention transferred to them are confirmed to maintain the extraneous DNA stably through crossbreeding, and they can be then bred generation after generation as animals carrying said DNA under normal breeding environments. Further, the desired extraneous DNA can be used as a starting material by integrating it in the plasmid described above. The DNA construct having the promoter can be produced in usual DNA engineering means. The abnormal DNA of the present invention is transferred to cells at the stage of fertilized egg cells so as to secure the presence of the DNA in all embryonic cells and somatic cells of the intended mammals. The presence of the abnormal DNA of the present invention in the embryonic cells in the produced animals after transfer of the DNA means that the abnormal DNA of the present invention is maintained in all embryonic cells and somatic cells in the offspring of the produced animals. The offspring of the produced animals has inherited the abnormal DNA of the present invention so that the abnormal DNA of the present invention is present in all embryonic cells and somatic cells of the offspring. Homozygote animals having the introduced DNA in both homogenous chromosomes are obtained, and these male and female animals are crossbred to produce the offspring having said DNA and capable of breeding generation after generation.
   The non-human mammals having the abnormal DNA of the present invention can be used as a morbid animal model because the abnormal DNA of the present invention is highly expressed in these animals to inhibit the functions of endogenous normal DNA, resulting in the onset of functional inactivated refractory conditions of the protein of the present invention. For example, the abnormal DNA-transferred animals of the present invention can be used to elucidate the mechanism of the morbid functional inactivated refractory conditions of the protein of the present invention or to examine the method of treating this disease.
   For specific applicability, the abnormal DNA high-expression animals of the present invention serve as a model for elucidation of the functional inhibition (dominant negative action) on the normal protein by the abnormal protein of the present invention in the functional inactivated refractory conditions of the protein of the present invention.
   Further, the mammals having the extraneous abnormal DNA of the present invention transferred to them can also be used in a test for screening a treating agent against the functional inactivated refractory conditions of the protein of the present invention because these animals have the symptom of an increase in the free protein of the present invention.
   Other applicability of the two DNA-transfected animals of the present invention includes:
   1. Use as a cellular source for tissue culture;
   2. Analysis of the relationship of the DNA with proteins specifically expressed or activated by the protein of the present invention by direct analysis of DNA or RNA in tissues in the DNA-transferred animals of the present invention or by analysis of tissues where the protein is expressed by the DNA;
   3. Study of the functions of cells from tissues generally difficult to culture, by using cultured cells of tissues having the DNA by standard tissue culture techniques;
   4. Screening for pharmaceutical agents for raising the functions of cells by using the cells of the above item 3; and
   5. Isolation and purification of the mutated protein of the present invention and preparation of an antibody against said protein.

   Further, the DNA-transferred animals of the present invention can be used to examine the clinical symptoms of diseases connected with the protein of the present invention, including the functional inactivated refractory conditions of the protein of the present invention, to obtain detailed pathological findings in each organ in a model with diseases connected with the protein of the present invention, and to contribute to development of new therapeutic methods as well as to the study and treatment of secondary diseases resulting from said diseases.
   Further, each organ is removed from the DNA-transferred animals of the present invention, cut into thin pieces, and digested with proteinase such as trypsin, whereby the free DNA-transferred cells can be obtained, and these cells can be cultured and these cultured cells can be established as a cell line. By identifying those cells producing the protein of the present invention and by examining their relationship with apoptosis, differentiation or multiplication or the signal transfer mechanism therein, their abnormalities can be examined so that these cells can be used as an useful material for study on the protein of the present invention and in elucidating their action.
   The DNA-transferred animals of the present invention can also be used to provide an effective and rapid screening method for treating agents for diseases connected with the protein of the present invention by use of the examination method and quantification method described above, so that the treating agents for these diseases including the functional inactive type refractory conditions of the protein of the present invention can be developed. Further, the DNA-transferred animals of the present invention or the extraneous DNA expression vector of the present invention can be used for examination and development of the DNA therapeutic method to treat diseases connected with the protein of the present invention.
(9) Knock-out animal
   The present invention provides a non-human mammalian embryonic stem cell wherein the DNA of the present invention is inactivated, as well as a non-human mammal deficient in expression of the DNA of the present invention.
   That is, the present invention provides:
   [1] A non-human mammalian embryonic stem cell wherein the DNA of the present invention is inactivated;
   [2] The embryonic stem cell according to the above [1], wherein the DNA is inactivated by introducing a reporter gene (e.g., β-galactosidase gene derived from Escherichia coli);
   [3]The embryonic stem cell according to the above [1], which is resistant to neomycin;
   [4]The embryonic stem cell according to the above [1], wherein the non-human mammal is a rodent;
   [5] The embryonic stem cell according to the above [4], wherein the non-human mammal is a mouse;
   [6] A non-human mammal deficient in expression of the DNA of the present invention, wherein the DNA is inactivated;
   [7] The non-human mammal according to the above [6], wherein said DNA is inactivated by introducing a reporter gene (e.g., β- galactosidase gene derived from Escherichia coli), and said reporter gene can be expressed under the control of a promoter for the DNA of the present invention;
   [8] The non-human mammal according to the above [6], wherein the non-human mammal is a rodent;
   [9] The non-human mammal according to the above [8], wherein the rodent is a mouse; and
   [10] Amethod of screening for a compound or a salt thereof promoting or inhibiting the activity of a promoter for the DNA of the present invention, which comprises administering a test compound into the animal of the above [7] and detecting the expression of the reporter gene.

   The non-human mammalian embryonic stem cells wherein the DNA of the present invention is inactivated refer to embryonic stem cells (referred to hereinafter as ES cells) of non-human mammals wherein the DNA does substantially not possess an ability to express the protein of the present invention (said DNA is also referred to hereinafter as the nock-out DNA of the present invention) by artificially mutating the DNA of the present invention possessed by the non-human mammals, thus suppressing the ability of the DNA to be expressed, or by substantially eliminating the activity of the protein of the present invention encoded by said DNA.
   As the non-human mammals, those described above are used.
   The DNA of the present invention can be artificially mutated by eliminating a part or the whole of said DNA sequence in genetic engineering means, by inserting another DNA thereto, or by substitution thereof. By these mutations, the reading frame of codons is shifted or the functions of the promoter or exons are destroyed whereby the knock-out DNA of the present invention can be produced.
   The non-human mammalian embryonic stem cells wherein the DNA of the present invention is inactivated (referred to hereinafter as the DNA-inactivated ES cells of the present invention or the nock-out ES cells of the present invention) can be obtained as follows. For example, the DNA of the present invention possessed by the desired non-human mammals is isolated, and the functions of its exons are destroyed by inserting a chemical resistance gene such as a neomycin resistance gene, a hygromycin resistance gene or a reporter gene such as lacZ (β-galactosidase gene), cat (chloramphenicol acetyl transferase gene) etc. into the exons, or the gene is destroyed by inserting a DNA sequence (e.g., poly A-added signal) for terminating translation of the gene into introns between exons to prevent synthesis of complete messenger RNA, and a DNA chain (referred to hereinafter as targeting vector) having the DNA sequence thus constructed is inserted into the chromosome of the animal by homologous recombination, and the resulting ES cells are analyzed by Southern hybridization with the DNA of the present invention or with its neighboring DNA sequence as the probe or by PCR where the DNA sequence on the targeting vector and a DNA sequence adjacent to the DNA of the present invention used for preparing the target vector are used as primers, whereby the nock-out ES cells of the present invention can be selected and obtained.
   Further, the original ES cells, which inactivate the DNA of the present invention by homologous recombination, may be those previously established as described above, or may be those newly established by the known method of Evans and Kaufma. For example, 129 line ES cells are generally used as mouse ES cells, but because their immunological background is not elucidated and for the purpose of obtaining pure ES cells having an immunologically evident genetic background, BDF₁ mice (F₁ between C57BL/6 and DBA/2) established by crossing with DBA/2 in improving a small number of collected eggs of C57BL/6 mice and C57BL/6 can also be used advantageously. The BDF₁ mice are advantageous because of their production of a large number of strong eggs, and further because they are derived from C57BL/6 mice so that the ES cells obtained therefrom provide morbid model mice which upon back-crossing with C57BL/6 mice can be used in place of C57BL/6 mice for the same genetic background.
   For establishing the ES cells, blastocysts on Day 3.5 after fertilization are generally used, but blastocysts obtained by collecting and culturing embryos at the 8-cell stage can also be used for efficiently producing a large number of early embryos.
   Male or female ES cells may be used, but male ES cells are convenient for production of germ cell line chimera. To reduce the cumbersome operation for their culture, it is desired to sex the cells as early as possible.
   For judging whether the ES cells are male or female, there is a method of amplifying and detecting a gene in sex-determining region on the Y chromosome by PCR. By using this method, the number of necessary ES cells can be reduced to the number of cells (about 50 cells) in about one colony as opposed to the number (about 10⁶) of cells required for conventional analysis of karyotype, so that the primary selection of ES cells in an early stage of culture can be performed by sexing thereof, and selection of the male cells is feasible at an early stage so that the operation of culture at an early stage can be significantly reduced.
   The secondary selection can be carried out for example by confirmation of the number of chromosomes by the G-binding method. The number of chromosomes in the resulting ES cells is desirably normal (i.e. 100%), but if the normal number of chromosomes is hardly achieved because of the operation etc. for establishment of the cell line, it is desired that the gene of the ES cells is knocked out and then cloned again into normal cells (e.g., cells with the normal number of chromosomes (2n=40)).
   The growth ability of the embryonic stem cell line thus obtained is usually very good, but the cells easily lose individual development, and thus they should be carefully subcultured. For example, these cells are cultured at about 37 °C in a CO₂ incubator (preferably in 5% CO₂ gas and 95% air or 5% oxygen, 5% CO₂ gas and 90% air) in the presence of LIF (1-10000 U/ml) on feeder cells such as STO fibroblasts, and during subculture, the cells are divided into single cells by treatment with trypsin/EDTA solution (usually 0.001 to 0.5% trypsin/0.1 to 5 mM EDTA, preferably about 0.1% trypsin/1 mM EDTA) and plated onto freshly prepared feeder cells. Usually, such subculture is conducted every 1 to 3 days during which the cells are observed, and if morphologically abnormal cells are observed, the cultured cells are desirably discarded.
   The ES cells can be differentiated into various types of cells in parietal muscles, internal-organ muscles and cardiac muscles by single-layer culture until the cells grow at high density or by float-culture until cellular mass is formed (M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology and Experimental Morphology, 87, 27, 1985), and the cells deficient in expression of the DNA of the present invention obtained by differentiation of the ES cells of the present invention are useful in in vitro cellular biological examination of the protein of the present invention.
   The non-human mammals deficient in expression of the DNA of the present invention can be distinguished from normal animals by measuring the amount of mRNA in said animals by a known method and comparing its expression indirectly.
   As the non-human mammals, those described above are used.
   In the non-human mammals deficient in expression of the DNA of the present invention, the targeting vector constructed as described above is introduced into mouse embryonic stem cells or mouse egg cells, and the DNA sequence where the DNA of the present invention in the targeting vector is inactivated by introduction is subjected to genetic homologous recombination to cause homologous recombination for replacing it by the DNA of the present invention on the chromosome in the mouse stem embryonic cells or mouse egg cells, whereby the DNA of the present invention can be knocked out.
   The cells wherein the DNA of the present invention is knocked out can be judged by Southern hybridization with a DNA sequence on the DNA of the present invention or with its neighboring DNA sequence as the probe or by PCR where a DNA sequence on the targeting vector and a mouse-derived DNA sequence adjacent to the DNA of the present invention used in the targeting vector are used as primers. When the non-human mammalian embryonic stem cells are used, the cell line wherein the DNA of the present invention is inactivated is cloned by genetic homologous recombination, and the cells are introduced into, for example, non-human animal embryos or blastocysts at e.g. the 8-cell stage, and the prepared chimera embryos are transplanted in uterus in the pseudo-pregnant non-human mammals. The produced animals are chimera animals composed of both cells having the normal DNA locus of the present invention and the artificially mutated DNA locus of the present invention.
   When some reproductive cells in said chimera animals have the partially mutated DNA locus of the present invention, those individuals composed of cells having the DNA locus of the present invention, wherein all tissues are artificially mutated, can be obtained by, for example, judgment of coat color from a group of individuals obtained by crossbreeding such chimera animals with normal animals. The individuals thus obtained are usually those individuals deficient in hetero-expression of the protein of the present invention, and such individuals deficient in hetero-expression of the protein of the present invention are mutually crossbred, and from their offspring, those individuals deficient in homo-expression of the protein of the present invention can be obtained.
   When egg cells are used, the DNA solution is injected into nuclei of egg cells by microinjection, whereby transgenic non-human mammals having the targeting vector introduced into their chromosome can be obtained, and those animals having a mutation in the DNA locus of the present invention resulting from genetic homologous recombination are obtained by comparison with the above transgenic non-human mammals.
   The individuals wherein the DNA of the present invention is knocked out in this manner are crossbred, and their offspring also is conformed to have the knocked-out DNA, and these animals can be bred generation after generation under normal breeding environments.
   Further, their germ cell line can also be obtained and maintained in a usual manner. That is, those male and female animals having the inactivated DNA can be crossbred to produce homozygote animals having the inactivated DNA in both homologous chromosomes. The resulting homozygote animals can be obtained efficiently by breeding under such conditions that one normal individual and plural homozygote individuals are present for one female parent. By crossbreeding the male and female heterogenote animals, the homozygote and heterozygote animals having the inactivated DNA are bred generation after generation.
   The non-human mammalian embryonic stem cells wherein the DNA of the present invention is inactivated are very useful for production of the non-human mammals deficient in expression of the DNA of the present invention.
   Further, the non-human mammals deficient in expression of the DNA of the present invention are deficient in various biological activities inducible by the protein of the present invention, and thus these animals can serve as a model with those diseases attributable to the inactivation of the biological activity of the protein of the present invention, and thus they are useful for elucidation of the cause of these diseases and for examination of the method for treating these diseases.
(9a) A method of screening for a compound having a therapeutic or preventive effect on diseases attributable to defect or damage of the DNA of the present invention
   The non-human mammals deficient in expression of the DNA of the present invention can be used for screening for a compound having a therapeutic or preventive effect on diseases (e.g., immune diseases, pulmonary insufficiency, pancreatic insufficiency, infectious diseases or gastrointestinal disorders) attributable to the defect or damage of the DNA of the present invention.
   That is, the present invention provides a method of screening for a compound having a therapeutic or preventive effect on those diseases attributable to the defect or damage of the DNA of the present invention, which comprises administering a test compound into the non-human mammals deficient in expression of the DNA of the present invention and then observing and measuring a change in the animals.
   The non-human mammals deficient in expression of the DNA of the present invention used in the screening method include those described above.
   The test compound includes, for example, peptides, proteins, non-peptide compounds, synthetic compounds, fermentation products, cellular extracts, plant extracts, animal tissue extracts, plasma etc., and these compounds may be novel or known compounds.
   Specifically, the non-human mammals deficient in expression of the DNA of the present invention are treated with a test compound and compared with untreated control animals, and the changes in the conditions of each organ and tissues of the animals and in the conditions of their diseases can be examined as an indicator for the therapeutic or preventive effect of the test compound.
   The method of treating the test animals with a test compound includes, for example, an oral administration, an intravenous injection etc. which can be selected depending on the conditions of the test animal and the properties of the test compound. The dose of the test compound can be suitably selected depending on the administration method, the properties of the test compound, etc.
   For example, if a compound having a therapeutic or preventive effect on pancreatic insufficiency is screened, the mammals deficient in expression of the DNA of the present invention is treated by sugar loading, and a test compound is administered into the animals before and after the treatment by sugar loading, and the blood sugar level in the animals and the change in their weight are measured with time.
   The compound obtained in the screening method is a compound selected from the test compounds described above, and has a therapeutic or preventive effect on the diseases (e.g., immune diseases, pulmonary insufficiency, pancreatic insufficiency, infectious diseases or gastrointestinal disorders) attributable to the defect or damage of the protein etc. of the present invention, and thus it can be used as safe and low-toxic pharmaceutical preparation against the diseases. The compound derived from the compounds obtained in the screening method described above can also be used in analogous manner.
   The compound obtained in the screening method may form a salt. Such salts of said compound are in the form of salts with physiologically acceptable acids (e.g., inorganic acids, organic acids) or bases (e.g., alkali metal salts), among which physiologically acceptable acid-addition salts are preferable. Such salts include, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid).
   A pharmaceutical preparation comprising the compound or a salt thereof, obtained in said screening method, can be produced in the same manner as in production of the above-described pharmaceutical preparation comprising the protein of the present invention.
   Since the pharmaceutical preparation thus obtained is safe and low toxic, it can be administered into humans or mammals (e.g., rat, mouse, guinea pig, rabbit, sheep, pig, cow, horse, cat, dog, monkey etc.).
   The dose of said compound or a salt thereof is varied depending on the intended diseases, the subject of administration, administration route etc., and when the compound is orally administered for the purpose of treatment of inflammatory diseases, said compound is administered in a dose of usually about 0.1 to 100 mg, preferably about 1.0 to 50 mg and more preferably about 1.0 to 20 mg every day into a (60 kg) adult. For parenteral administration, the dose of said compound is varied depending on the subject of administration, the intended diseases etc., and when the compound is administered in the form of an injection for the purpose of treatment of inflammatory diseases, said compound is conveniently injected intravenously in a dose of usually about 0.01 to 30 mg, preferably about 0.1 to 20 mg and more preferably about 0.1 to 10 mg every day into a (60 kg) adult. Said compound can be administered into other animals in the same dose (per 60 kg) as described above.
(9b) A method of screening for a compound promoting or inhibiting the activity of a promoter for the DNA of the present invention
   The present invention provides a method of screening for a compound or a salt thereof promoting or inhibiting the activity of a promoter for the DNA of the present invention, which comprises administering a test compound into the non-human mammals deficient in expression of the DNA of the present invention and then detecting expression of the reporter gene.

The non-human mammals deficient in expression of the DNA of the present invention in the screening method described above make use of those non-human mammals deficient in expression of the DNA of the present invention described above, wherein the DNA of the present invention is inactivated by introducing a reporter gene and said reporter gene is expressed under the control of a promoter for the DNA of the present invention.

The test compound includes those described above.

The reporter gene includes those described above, and β - galactosidase gene (lacZ), soluble alkali phosphatase gene or luciferase gene is preferable.

In the non-human mammals deficient in expression of the DNA of the present invention wherein the DNA of the present invention is replaced by the reporter gene, the reporter gene is present under the control of the promoter for the DNA of the present invention, and thus the activity of the promoter can be detected by tracing expression of a substance encoded by the reporter gene.

For example, if a part of the DNA region encoding for the protein of the present invention has been replaced by β - galactosidase gene (lacZ) derived from Escherichia coli, β -galactosidase is expressed in place of the protein of the present invention in tissues which originally expressed the protein of the present invention. Accordingly, a reagent serving as β - galactosidase substrate such as 5-bromo-4-chloro-3-indolyl-β -galactopyranoside (X-gal) is used for staining, whereby the state of expression of the protein of the present invention in vivo in the animals can be easily observed. Specifically, the mouse deficient in the protein of the present invention, or their tissue section, is fixed with glutaraldehyde and then washed with phosphate buffered saline (PBS) and allowed to react in a staining solution containing X-gal at room temperature or at about 37 °C for about 30 minutes to 1 hour, and the β-galactosidase reaction is terminated by washing the tissue specimen with 1 mM EDTA/PBS, and its coloration is observed. Alternatively, mRNA encoding for lacZ may be detected in a usual manner.

The compound or a salt thereof obtained by the screening method described above is a compound selected from the test compounds described above and promotes or inhibits the activity of the promoter for the DNA of the present invention.

The compound obtained in the screening method may form salts, and the salts of said compound are in the form of salts with physiologically acceptable acids (e.g., inorganic acids) or bases (e.g., organic acids), among which physiologically acceptable acid-addition salts are preferable. Such salts include, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The compounds or a salt thereof promoting the promoter activity for the DNA of the present invention can promote expression of the protein of the present invention and can promote the functions of said protein, and thus they are useful as safe and low toxic treating or preventing agents against diseases such as immune diseases, pulmonary insufficiency, pancreatic insufficiency, infectious diseases or gastrointestinal disorders.

Further, compounds derived from the compounds obtained in the screening method described above can be used in analogous manner.

A pharmaceutical preparation comprising the compound or a salt thereof, obtained in the screening method described above, can be produced in the same manner as in production of the pharmaceutical preparation comprising the protein or a salt thereof of the present invention described above.

Since the pharmaceutical preparation thus obtained is safe and low toxic, it can be administered into humans or mammals (e.g., rat, mouse, guinea pig, rabbit, sheep, pig, cow, horse, cat, dog, monkey etc.).

The dose of said compound or a salt thereof is varied depending on the intended diseases, the subject of administration, administration route etc., and when the compound promoting the promoter activity for the DNA of the present invention is orally administered for the purpose of treatment of inflammatory diseases, said compound is administered in a dose of usually about 0.1 to 100 mg, preferably about 1.0 to 50 mg and more preferably about 1.0 to 20 mg every day into a (60 kg) adult. For parenteral administration, the dose of said compound is varied depending on the subject of administration, the intended diseases etc., and when the compound promoting the promoter activity for the DNA of the present invention is administered in the form of an injection for the purpose of treatment of inflammatory diseases, said compound is conveniently injected intravenously in a dose of usually about 0.01 to 30 mg, preferably about 0.1 to 20 mg and more preferably about 0.1 to 10 mg every day into a (60 kg) adult. Said compound can be administered into other animals in the same dose (per 60 kg) as described above.

On the other hand, when the compound inhibiting the promoter activity for the DNA of the present invention is orally administered, said compound is administered in a dose of usually about 0.1 to 100 mg, preferably about 1.0 to 50 mg and more preferably about 1.0 to 20 mg every day into a (60 kg) adult. For parenteral administration, the dose of said compound is varied depending on the subject of administration, the intended diseases etc., and when the compound inhibiting the promoter activity for the DNA of the present invention is administered in the form of an injection, said compound is conveniently injected intravenously in a dose of usually about 0.01 to 30 mg, preferably about 0.1 to 20 mg and more preferably about 0.1 to 10 mg every day into a (60 kg) adult. Said compound can be administered into other animals in the same dose (per 60 kg) as described above.

The non-human mammals deficient in expression of the DNA of the present invention are very useful for screening for a compound or a salt thereof, which promotes or inhibits the activity of the promoter for the DNA of the present invention, and can contribute significantly to elucidation of the causes of various diseases attributable to deficient expression of the DNA of the present invention or to development of preventing or treating agents.

When bases and amino acids are represented by abbreviation in the present specification and drawings, it is based on abbreviation according to IUPAC-IUB Commision on Biochemical Nomenclature or the conventional abbreviation in the art and, examples thereof are described below. If amino acids can occur as optical isomers, L-isomers are referred to unless otherwise specified.
- DNA:: deoxyribonucleic acid
- cDNA:: complementary deoxyribonucleic acid
- A:: adenine
- T:: thymine
- G:: guanine
- C:: cytosine
- RNA:: ribonucleic acid
- mRNA:: messenger ribonucleic acid
- dATP:: deoxyadenosine triphosphate
- dTTP:: deoxythymidine triphosphate
- dGTP:: deoxyguanosine triphosphate
- dCTP:: deoxycytidine triphosphate
- ATP:: adenosine triphosphate
- EDTA:: ethylene diamine tetraacetate
- SDS:: sodium dodecyl sulfate
- Gly:: glycine
- Ala:: alanine
- Val:: valine
- Leu:: leucine
- Ile:: isoleucine
- Ser:: serine
- Thr:: threonine
- Cys:: cysteine
- Met:: methionine
- Glu:: glutamic acid
- Asp:: aspartic acid
- Lys:: lysine
- Arg:: arginine
- His:: histidine
- Phe:: phenylalanine
- Tyr:: tyrosine
- Trp:: tryptophan
- Pro:: proline
- Asn:: asparagine
- Gln:: glutamine
- pGlu:: pyroglutamic acid

The substituent groups, protecting groups and reagents appearing frequently in the present specification are expressed in the following symbols.
- Me:: methyl group
- Et:: ethyl group
- Bu:: butyl group
- Ph:: phenyl group
- TC:: thiazolidine-4 (R)-carboxamido group
- Tos:: p-toluene sulfonyl
- CHO:: formyl
- Bzl:: benzyl
- Cl₂-Bzl:: 2,6-dichlorobenzyl
- Bom:: benzyloxymethyl
- Z:: benzyloxycarbonyl
- Cl-Z:: 2-chlorobenzyloxycarbonyl
- Br-Z:: 2-bromobenzyloxycarbonyl
- Boc:: t-butoxycarbonyl
- DNP:: dinitrophenyl
- Trt:: trityl
- Bum:: t-butoxymethyl
- Fmoc:: N-9-fluorenyl methoxycarbonyl
- HOBt:: 1-hydroxybenztriazole
- HOOBt:: 3,4-dihydro-3-hydroxy-4-oxo-1,2,3-benzotriazine
- HONB:: 1-hydroxy-5-norbornene-2,3-dicarboximide
- DCC:: N,N'-dicyclohexyl carbodiimide

The sequence numbers in the Sequence Listing in the present specification show the following sequences:
SEQ ID NO: 1 shows an amino acid sequence of the human-derived protein of the present invention.
SEQ ID NO: 2 shows an amino acid sequence of the rat-derived protein of the present invention.
SEQ ID NO: 3 shows an amino acid sequence of the mouse-derived protein of the present invention.
SEQ ID NO: 4 shows a base sequence of DNA encoding for the human-derived protein of the present invention having the amino acid sequence represented by SEQ ID NO: 1.
SEQ ID NO: 5 shows a base sequence of DNA encoding for the rat-derived protein of the present invention having the amino acid sequence represented by SEQ ID NO: 2.
SEQ ID NO: 6 shows a base sequence of DNA encoding for the mouse-derived protein of the present invention having the amino acid sequence represented by SEQ ID NO: 3.
SEQ ID NO: 7 shows an amino acid sequence of a partial peptide of the human-derived protein of the present invention. This corresponds to an amino acid sequence at the 23 to 119 positions in the amino acid sequence represented by SEQ ID NO: 1.
SEQ ID NO: 8 shows an amino acid sequence of a partial peptide of the rat-derived protein of the present invention. This corresponds to an amino acid sequence at the 23 to 119 positions in the amino acid sequence represented by SEQ ID NO: 2.
SEQ ID NO: 9 shows an amino acid sequence of a partial peptide of the mouse-derived protein of the present invention. This corresponds to an amino acid sequence at the 23 to 119 positions in the amino acid sequence represented by SEQ ID NO: 3.
SEQ ID NO: 10 shows the base sequence of DNA encoding for the partial peptide of the present invention having the amino acid sequence represented by SEQ ID NO: 7.
SEQ ID NO: 11 shows the base sequence of DNA encoding for the partial peptide of the present invention having the amino acid sequence represented by SEQ ID NO: 8.
SEQ ID NO: 12 shows the base sequence of DNA encoding for the partial peptide of the present invention having the amino acid sequence represented by SEQ ID NO: 9.
SEQ ID NO: 13 shows the base sequence of the primer M440-OF used in Example 4.
SEQ ID NO: 14 shows the base sequence of the primer H440-EF used in Example 6.
SEQ ID NO: 15 shows the base sequence of the primer H440-ER used in Example 6.
SEQ ID NO: 16 shows the base sequence of the primer H440-OF used in Example 6.
SEQ ID NO: 17 shows the base sequence of the primer H440-OR used in Example 6.
SEQ ID NO: 18 shows the base sequence of the primer R440-OF used in Example 6.
SEQ ID NO: 19 shows the base sequence of the primer R440-OR used in Example 6.

The transformant Escherichia coli XL1-Blue MRF' /pDRL440H obtained in Example 6 below has been deposited under Accession Number of FERM BP-6476 since August 26, 1998 with at National Institute of Bioscience and Human-Technology (NIBH), Agency of Industrial Science & Technology, Ministry of International Trade & Industry, and under Accession Number of IFO 16192 with Institute for Fermentation, Osaka, Japan (IFO) since July 31, 1998.

The transformant Escherichia coli XL1-Blue MRF' /pDRL440M obtained in Example 4 below has been deposited under Accession Number of FERM BP-6477 since August 26, 1998 with at National Institute of Bioscience and Human-Technology (NIBH), Agency of Industrial Science & Technology, Ministry of International Trade & Industry, and under Accession Number of IFO 16193 with Institute for Fermentation, Osaka, Japan (IFO) since July 31, 1998.

The transformant Escherichia coli XL1-Blue MRF' /pDRL440R obtained in Example 6 below has been deposited under Accession Number of FERM BP-6478 from August 26, 1998 with National Institute of Bioscience and Human-Technology (NIBH), Agency of Industrial Science & Technology, Ministry of International Trade & Industry, and under Accession Number of IFO 16194 with Institute for Fermentation, Osaka, Japan (IFO) since July 31, 1998.

Hereinafter, the present invention is described in more detail by reference to the Reference Example and the Examples, but these are not intended to limit the present invention. The genetic manipulation using Escherichia coli was conducted in accordance with methods described in Molecular Cloning.

### Reference Example 1

### Construction of animal cell expression vector pCAN618

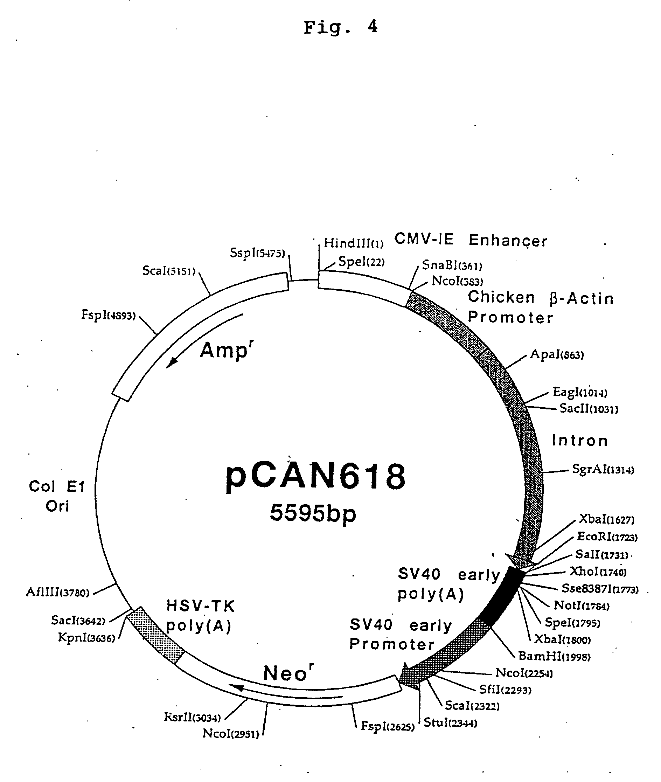

Plasmid pBK/CMV (4512 bp) having a neomycin resistance gene downstream from an SV40 early gene promoter (Stratagene) was digested with Bsu36I (New England Biolabs), and the resulting 1.6 kbp fragment was treated with DNA polymerase I Klenow fragment (Takara Shuzo) to give a blunt-ended fragment containing the neomycin resistance gene. This fragment was ligated with a plasmid obtained by digesting pME18S (3411 bp) with SmaI (Takara Shuzo) to give pME18S/Neo (5040 bp). Then, a plasmid having a HindIII linker (Takara Shuzo) introduced to HincII site of pCXN2 having a β-actin promoter in a region downstream from a very early gene enhancer from cytomegalovirus was digested with the two restriction enzymes HindIII (Takara Shuzo) and EcoRI (Takara Shuzo) to give a 1.7 kb fragment containing the β-actin promoter downstream from the very early gene enhancer from cytomegalovirus. This fragment was ligated with a 4.2 kbp fragment obtained by digesting pME18S/Neo with the two restriction enzymes HindIII and EcoRI, to give an animal cell expression vector pCAN616 (5969 bp). Further, pCAN616 was digested with XhoI (Takara Shuzo) and then self-closed to give animal cell expression vector pCAN617 (5585 bp) from which a stuffer region in a multi-cloning site had been removed. On the other hand, pCAN616 was digested with two enzymes EcoRI and XhoI to give a 5.6 kb fragment which was then ligated with a 17-mer synthetic oligonucleotide containing an EcoRI-SalI-XhoI site to give animal cell expression vector pCAN618 (5595 bp) (Fig. 4).

### Example 1

### Selection of clones from data base and analysis of base sequence

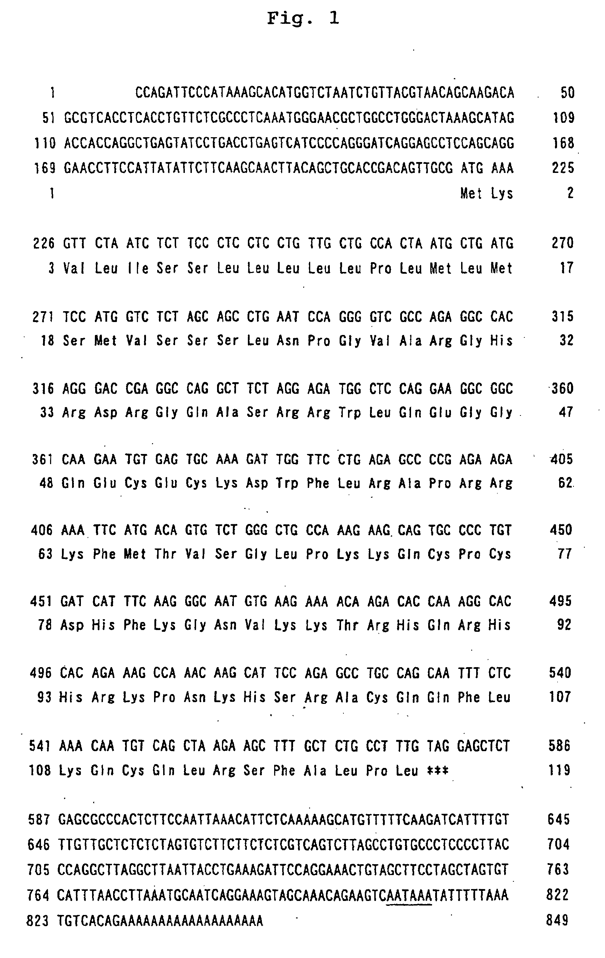

Clones encoding for a processing site and a signal sequence for secretion, out of an EST database available from Smith Kline Beecham (SB), were selected. The DNA sequence of EST was translated into an amino acid sequence, and a clone having a cluster of hydrophobic amino acids (Leu, Ile, Val, Phe, Ala etc.) after Met and having a processing site (ArgArg, LysArg, LysLys) in the same frame was selected, and as a clone satisfying these requirements, HGS: 105111 was found. However, the EST sequence in the database may have usually a deletion, insertion and misreading therein, and thus the sequence was confirmed in the following method. This clone was obtained as TGC-440 from SB, and the plasmid DNA was digested with EcoRI and XhoI, and the size of the inserted DNA fragment was analyzed by 1.2% agarose gel electrophoresis, and as a result, it was found to be 0.85 kb cDNA fragment. Further, T7 primer and T3 primer were used to analyze base sequences of the inserted DNA fragment by a fluorescent DNA sequencer (Perkin Elmer; ABI PRISM 377 DNA sequence). This DNA sequence and an amino acid sequence deduced therefrom are shown in Fig. 1. It was revealed that the region encoding for the protein is present at the 220 to 576 positions in the sequence in Fig. 1.

### Example 2

### Analysis of the expression site

20 ng of the insert DNA fragment (EcoRI-XhoI, 0.85 kb fragment) described in Example 1 and 5 µl [α-³²P] dCTP (Amersham: 6000 Ci/mmol) were used to prepare a DNA probe by the method of Multiprime DNA labeling system (Amersham; PRN. 1601Y). By Northern blotting with this probe, human multi-tissue Northern blots (Clontech; #7759-1 & #7760-1) were analyzed. The conditions for hybridization and washing were those in accordance with the manufacture's instructions, and the detection was performed with BAS-2000 (Fuji Film). As a result, it was revealed that the mRNA of this clone is expressed in limited tissues such as human lungs, trachea and stomach, and the mRNA was found to be an organ-specific expression product. Further, it was also revealed that the size of the mRNA is as short as about 0.8 kb and 0.6 kb, and the cDNA fragment of TGC-440 contains almost the full length mRNA, and it was also revealed that the coding region for the protein was within the region shown in Fig. 1.

### Example 3

### Obtaining rat cDNA

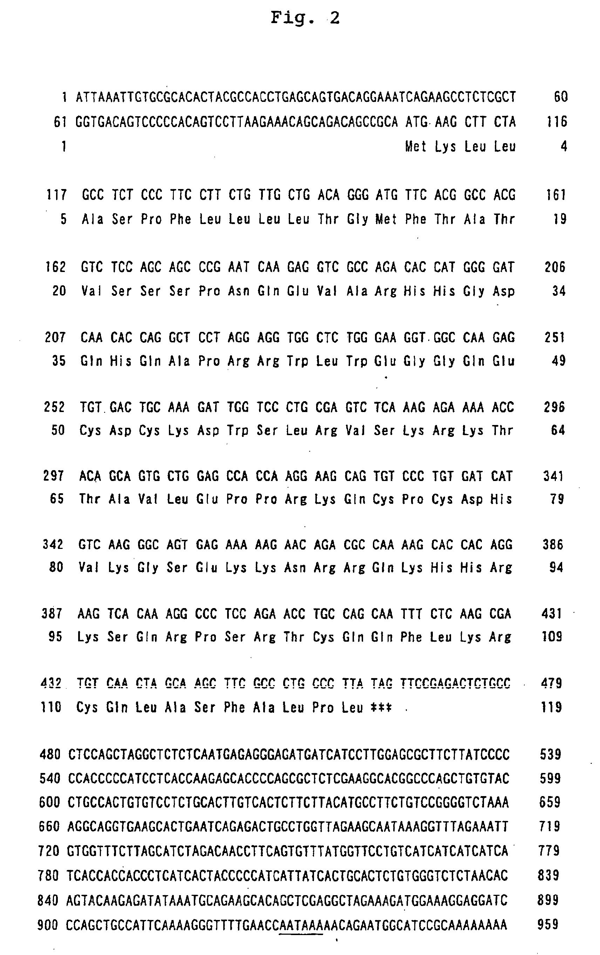

Lungs were excised from two SD rats, and RNA was extracted form the lungs by the per se known guanidine hydrochloride method and separated by use of an oligo (dT) cellulose column (Amersham) to give 19 µg poly(A) ⁺RNA. Using 5 µg of this RNA as a template, cDNA was synthesized in the method of SUPERSCRIPT™ choice system (GIBCO BRL), and an EcoRI adaptor was added to the cDNA. 200 ng of the cDNA fragment was inserted to an EcoRI site of λgt11 and used to prepare a phage library by in vitro packaging. Using a DNA probe prepared in the method described in Example 2, plaque hybridization was carried out. Hybridization was carried out in 5×SSPE, 5×Denhardt's solution, and 0.5% SDS at 65 °C overnight and washing was carried out with 0.5xSSC, and 0.1% SDS at 50 °C. A plurality of positive plaques were obtained by autoradiography (-80 °C, 18 hours), and then single-plaque isolation was conducted, and the phage DNA was digested with BsiWI, and the resulting insert cDNA fragment was slub-cloned into an Acc 651 site of pUC118. By determination of the base sequence of said cDNA fragment, it was revealed that the fragment encodes rat TGC-440 consisting of the same number (119) of amino acids as in human. The resulting sequence had 78% (at the DNA level) and 63% (at the amino acid level) homology to the human coding region. Further, a typical signal sequence consisting of 22 amino acids was present at the N-terminal. The base sequence of rat TGC-440 and an amino acid sequence deduced therefrom are shown in Fig. 2.

### Example 4

### Obtaining mouse cDNA

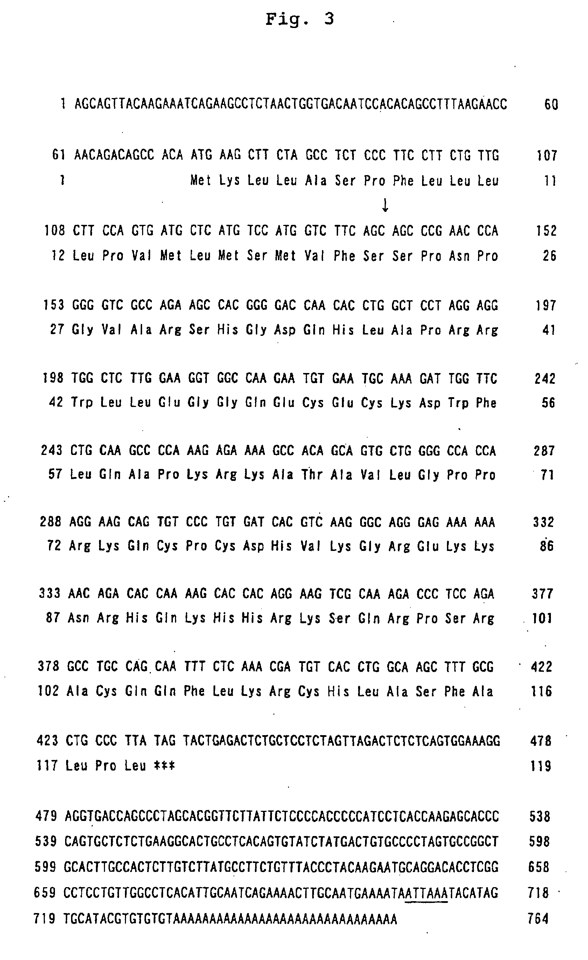

The lungs were excised from C57BL/6N mice, and poly(A)⁺ RNA was obtained in the same manner as in Example 3. Using this RNA as a template, mouse cDNA was obtained using 3'-RACE. Primer M440-OF (GCCTTTAAGAACCAACAGACAG; SEQ ID NO: 13) designed to amplify the open reading frame was used to conduct 3'-RACE in a usual manner. Said cDNA fragment (0.7 kb) was cloned into SrfI site in cloning vector pCR-Script Amp (Stratagene) to give pDRL440M (Escherichia coli XL1-Blue MRF' /pDRL440M). By sequencing the cDNA fragment, the primary structure of mouse TGC-440 consisting of the same number (119) of amino acids as in human and rat was revealed. At the N-terminal, a typical signal sequence consisting of 22 amino acids were present. The base sequence of mouse TGC-440 and an amino acid sequence deduced therefrom are shown in Fig. 3.

### Example 5

### Preparation of anti-TGC-440 antiserum

A peptide (human) consisting of an amino acid sequence at the 110 to 119 positions in the present protein represented by SEQ ID NO: 1, and a peptide consisting of an amino acid sequence at the 110 to 119 positions in the present protein (rat) represented by SEQ ID NO: 2, were chemically synthesized by a per se known method. Each 1 mg of these peptides was bound to 4 mg of bovine cyloglobulin by the maleimide method, and for first immunization, 100 µg antigen peptide along with FCA (complete Freund's adjuvant) was injected subcutaneously to 2 rabbits (SPF, New Zealand White), respectively. Thereafter, booster was conducted 3 times at 2-weeks intervals. In the second or subsequent booster, FIA (incomplete Freund's adjuvant) was used in place of FCA. In 1 week after final immunization, blood was collected from ear veins, and a serum fraction was obtained therefrom in a usual manner to give anti-TGC-440 antiserum.

### Example 6

### Construction of TGC-440 expression plasmid

Using primers, H440-EF (GACGAATTCCCACCATGAAAGTTCTAATCTCTTCCCTCCT; SEQ ID NO: 14) and H440-ER (GACTCGAGCGGCCGCTACAAAGGCAGAGCAAAGCTTCTTA; SEQ ID NO: 15) designed to amplify the open reading frame of human TGC-440 and synthesized in a per se known method, PCR was conducted where 1 ng of the plasmid described in Example 1 was used as the template. The conditions for PCR were that using Takara Ex Taq (Takara Shuzo), the PCR reaction was repeated 25 times at 95 °C for 30 seconds and 68 °C for 1 minute in a thermal cycler GeneAmp PCR System 2400 (Perkin Elmer). The resulting PCR fragment was digested with EcoRI and NotI and subcloned in EcoRI and NtI sites in the animal cell expression vector pCAN618 to give pCAN618/huTGC440.

Using primers, H440-OF (TGCACCGTCGACCACCATGAAAGTTCTAATCTCTTCCCTCCTCCTGT; SEQ ID NO: 16) and H440-OR (CGCTCAGTCGACCTACAAAGGCAGAGCAAAGCTTCTTAGCTGACATTGTTT; SEQ ID NO: 17) designed to amplify the open reading frame of human TGC-440 and synthesized in a per se known method, PCR was conducted where 1 ng of the plasmid described in Example 1 was used as the template. The conditions for PCR were that using Pfu DNA polymerase (Stratagene), the PCR reaction was repeated 25 times at 96 °C for 45 seconds, 54 °C for 45 seconds, and 72 °C for 1 minute in a thermal cycler GeneAmp PCR System 2400 (Perkin Elmer). The resulting PCR fragment was digested with SalI and subcloned in SalI site in the animal cell expression vector pA1-11 (also called pAKKO1.11) to give pDRL440H (Escherichia coli XL1-Blue MRF' /pDRL440H) having the cDNA in the normal direction to the promoter. Further, using primers R440-OF (ACAGCAGTCGACCACCATGAAGCTTCTAGCCTCTCCCTTCCTTCTGTTGCTGACAGGGA TGTTCAC; SEQ ID NO: 18) and R44-OR (CAGAGTGTCGACACTATAAGGGCAGGGCGAAGC; SEQ ID NO: 19) designed to amplify the open reading frame of human TGC-440 and synthesized in a per se known method, PCR was conducted where 1 ng of the plasmid described in Example 3 was used as the template. The conditions for PCR were that using Pfu DNA polymerase (Stratagene), and the PCR reaction was repeated 25 times at 96 °C for 45 seconds, 54 °C for 45 seconds, and 72 °C for 1 minute in a thermal cycler GeneAmp PCR System 2400 (Perkin Elmer). The resulting PCR fragment was digested with SalI and subcloned in SalI site in the animal cell expression vector pA1-11 (also called pAKKO1.11) to give pDRL440R (Escherichia coli XL1-Blue MRF' /pDRL440R) having the cDNA in the normal direction to the promoter.

### Example 7

### Expression of human TGC-440 cDNA in COS7 cells

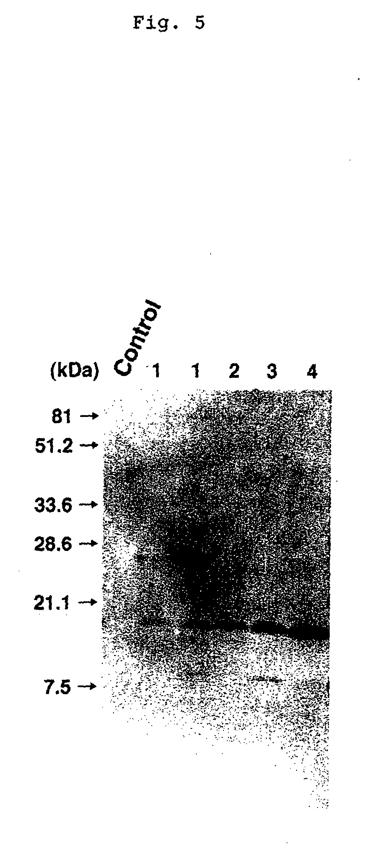

COS7 cells were cultured usually in DMEM medium (GIBCO-BRL) containing 10% FCS (fatal calf serum). The COS7 cells (1.5×10⁵ cells/well) were cultured in a 6-wells plate for 24 hours, then washed twice with Opti-MEM medium (GIBCO-BRL) and used to introduce an expression plasmid. 1 µg/well of the expression plasmid (pCAN618/huTGC440) obtained in Example 6 and 10 µl/well of Lipofect AMINE Reagent (GIBCO-BRL) were introduced into the COS7 cells in Opti-MEM serum-free medium according to the manufacture's instructions. In 5 hours after the expression plasmid was introduced, FCS was added thereto at a concentration of 10%, and the cells were further cultured for 19 hours, and thereafter, the medium was exchanged with (1) Opti-MEM medium (GIBCO-BRL), (2) Opti-MEM medium (GIBCO-BRL) containing 0.25 mM ABSF (Wako Pure Chemical Industries, Ltd.), (3) Opti-MEM medium (GIBCO-BRL) containing 0.05% CHAPS (Dojin Kagaku), or (4) Opti-MEM medium (GIBCO-BRL) containing 0.25 mM ABSF (Wako Pure Chemical Industries, Ltd.) and 0.05% CHAPS (Dojin Kagaku), and the cells were further cultured for 48 hours. The culture supernatant and cells were recovered separately and used for analysis by Western blotting. 500 µl of the supernatant was concentrated into 50 µl (10-fold conc.) by Microcon 3 (cut-off molecular weight: 3000) (Amicon). The cells were washed with physiological saline, and 200 µl of Laemmli sample buffer was added thereto and heated at 95 °C for 2 minutes to give a cell extract. The concentrated culture supernatant and the cell extract were electrophoresed by SDS polyacrylamide gel (18%, TEFCO) and transferred onto a nitrocellulose membrane (Hybond ECL, Amersham). The membrane was blocked with a blocking solution (50% Block Ace (Snow Brand Milk Products Co., Ltd.), 0.9% NaCl, and 20 mM Tris-HCl (pH 7.5)) for 1 hour and then allowed to react at room temperature for 2 hours with anti-TGC-440 antiserum diluted 2000-fold with 10% Block Ace/TBS-T (0.9% NaCl, 20 mM Tris-HCl (pH 7.5), and 0.05% Tween 20). It was washed 5 times with TBS-T and then allowed to react at room temperature for 1 hour with anti-rabbit IgG antibody (Amersham) labeled with horse radish peroxidase (HRP) diluted 4000-fold with 10% Block Ace/TBS-T. It was washed 5 times with TBS-T and then detected by chemoluminescence using ECL-plus Western blotting detection reagent (Amersham) on Superfilm ECL (Amersham). As a result, about 1.3 kDa and 1.1 kDa products were detected in the cell extract as shown in Fig. 5. Further, about 1.1 kDa product was detected in the culture supernatant, and it was revealed that TGC-440 was secreted extracellularly and present as a humoral factor.

### Example 8

### Establishment of CHO cell line constitutively secreting a large amount of human and rat TGC 440 protein

CHO (dhfr⁻) (ATCC) cells were cultured in α-MEM medium (Gibco-BRL) containing 10% FCS (Hyclone) at 37 °C in 5% CO₂ gaseous phase.

The CHO (dhfr⁻) (ATCC) cells were plated onto a 10-cm plate, then cultured for 24 hours and transfected with 10 µg each of the human and rat TGC440 expression plasmids obtained in Example 6 (human: pDRL440H; rat: pDRL440R) by the calcium phosphate method. After 12 hours, the medium was exchanged with α-MEM medium containing 10% FCS, and after 12 hours with a selective medium (α-MEM containing 10% dialyzed serum (Hyclone) without ribonucleotide and deoxyribonucleotide (Gibco-BRL)). Thereafter, the selective medium was exchanged at 3-days intervals, and those cells having the expression plasmid integrated therein were selected. On the ninth day after the selective medium was exchanged, the human and rat clones, each 12 colonies, grown on the plate were cloned and inoculated onto 12-wells plates.

Then, these clones were cultured until they became confluent and then the medium was exchanged with 1 ml of (1) α-MEM medium (Gibco-BRL), (2) α-MEM medium (Gibco-BRL) containing 0.25 mM ABSF (Wako Pure Chemical Industries. Ltd.), (3) α-MEM medium (Gibco-BRL) containing 0.05% CHAPS (Dojin Kagaku), or (4) α-MEM medium (Gibco-BRL) containing 0.25 mM ABSF (Wako Pure Chemical Industries, Ltd.) and 0.05% CHAPS (Dojin Kagaku), and the cells were further cultured for 24 hours. The culture supernatant was transferred to an eppendorf sample tube and centrifuged, and the floating cells were removed, and the supernatant was concentrated into 1/10 by centricon-3 (Amicon), and an equal volume of SDS-PAGE sample buffer containing DTT was added to each concentrate which was then subjected to SDS-PAGE, and the expression level was estimated by Western blotting. Western blotting was conducted using the anti-TGC440 antiserum (diluted 1/200-fold) obtained in Example 5 as the primary antibody and HRP-labeled anti-rabbit IgG antibody (1/2000, Amersham) as the second antibody and development was performed using an ECL Western blotting kit (Amersham).

As shown in Fig. 6 (human TGC 440 protein) and Fig. 7 (rat TGC 40 protein), it was confirmed that TGC440 protein having the same molecular weight as that expressed by COS-7 cells was expressed and secreted in the medium, and it was found that TCG 440 protein was expressed most in #5, 9 and 10 clones among the human TCG 440 protein-expressing CHO cells, and in #4 and 9 clones among the rat TGC 440 protein-expressing CHO cells. Further, these clones were plated onto a 96-well plate to give single-cell clones by limiting dilution.

### Industrial Applicability

The protein of the present invention and the DNA encoding for the same can be used for a treating or preventing agent for various diseases such as immune diseases, pulmonary insufficiency, pancreatic insufficiency, infectious diseases or gastrointestinal disorders. Further, the protein of the present invention is useful as a reagent for screening for a compound or a salt thereof which promotes or inhibits the activity of the protein of the present invention. In addition, the antibody against the protein of the present invention can specifically recognize the protein of the present invention and can thus be used for quantification of the protein of the present invention in a test solution.

## Claims

1. A protein which comprises an amino acid sequence identical or substantially identical with the amino acid sequence represented by SEQ ID NO: 1, SEQ ID NO: 2 or SEQ ID NO: 3, or a salt thereof.

2. A partial peptide of the protein of Claim 1, or a salt thereof.

3. A method for producing the protein of Claim 1 or the partial peptide of Claim 2, or a salt thereof, which comprises culturing a transformant transformed with a recombinant vector comprising DNA encoding for the protein of Claim 1 or the partial peptide of Claim 2 and forming the protein or the partial peptide.

4. An antibody against the protein of Claim 1 or the partial peptide of Claim 2, or a salt thereof.

5. A method of screening for a compound or a salt thereof promoting or inhibiting the activity of the protein of Claim 1 or the partial peptide of Claim 2, or a salt thereof, which comprises use of the protein of Claim 1 or the partial peptide of Claim 2, or a salt thereof.

6. A kit for screening for a compound or a salt thereof promoting or inhibiting the activity of the protein of Claim 1 or the partial peptide of Claim 2, or a salt thereof, which comprises the protein of Claim 1 or the partial peptide of Claim 2, or a salt thereof.

7. A compound or a salt thereof promoting or inhibiting the activity of the protein of Claim 1 or the partial peptide of Claim 2, or a salt thereof, which can be obtained by use of the method of screening of Claim 5 or the kit for screening of Claim 6.

8. A pharmaceutical preparation which comprises a compound or a salt thereof promoting or inhibiting the activity of the protein of Claim 1 or the partial peptide of Claim 2, or a salt thereof, which can be obtained by use of the method of screening of Claim 5 or the kit for screening of Claim 6.

9. A diagnostic agent which comprises the antibody of Claim 4.

10. A pharmaceutical preparation which comprises the antibody of Claim 4.


SEQUENCE LISTING
<110> Takeda Chemical Industries, Ltd.
<120> Novel Protein and its Production
<130> A98132
<150> JP 10-250108
<151> 1998-09-03
<160> 19
<210> 1
<211> 119
<212> PRT
<213> Human
<400> 1
Met Lys Val Leu Ile Ser Ser Leu Leu Leu Leu Leu Pro Leu Met Leu
1               5                   10                  15
Met Ser Met Val Ser Ser Ser Leu Asn Pro Gly Val Ala Arg Gly His
            20                  25                  30
Arg Asp Arg Gly Gln Ala Ser Arg Arg Trp Leu Gln Glu Gly Gly Gln
        35                  40                  45
Glu Cys Glu Cys Lys Asp Trp Phe Leu Arg Ala Pro Arg Arg Lys Phe
    50                  55                  60
Met Thr Val Ser Gly Leu Pro Lys Lys Gln Cys Pro Cys Asp His Phe
65                  70                  75                  80
Lys Gly Asn Val Lys Lys Thr Arg His Gln Arg His His Arg Lys Pro
                85                  90                  95
Asn Lys His Ser Arg Ala Cys Gln Gln Phe Leu Lys Gln Cys Gln Leu
            100                 105                 110
Arg Ser Phe Ala Leu Pro Leu
        115             119
<210> 2
<211> 119
<212> PRT

<213> Rat

<400> 2

Met Lys Leu Leu Ala Ser Pro Phe Leu Leu Leu Leu Thr Gly Met Phe
1               5                   10                  15

Thr Ala Thr Val Ser Ser Ser Pro Asn Gln Glu Val Ala Arg His His
            20                  25                  30

Gly Asp Gln His Gln Ala Pro Arg Arg Trp Leu Trp Glu Gly Gly Gln
        35                  40                  45

Glu Cys Asp Cys Lys Asp Trp Ser Leu Arg Val Ser Lys Arg Lys Thr
    50                  55                  60

Thr Ala Val Leu Glu Pro Pro Arg Lys Gln Cys Pro Cys Asp His Val
65                  70                  75                  80

Lys Gly Ser Glu Lys Lys Asn Arg Arg Gln Lys His His Arg Lys Ser
                85                  90                  95

Gln Arg Pro Ser Arg Thr Cys Gln Gln Phe Leu Lys Arg Cys Gln Leu
            100                 105                 110

Ala Ser Phe Ala Leu Pro Leu
        115             119

<210> 3

<211> 119

<212> PRT

<213> Mouse

<400> 3

Met Lys Leu Leu Ala Ser Pro Phe Leu Leu Leu Leu Pro Val Met Leu
1               5                   10                  15

Met Ser Met Val Phe Ser Ser Pro Asn Pro Gly Val Ala Arg Ser His
            20                  25                  30

Gly Asp Gln His Leu Ala Pro Arg Arg Trp Leu Leu Glu Gly Gly Gln
        35                  40                  45

Glu Cys Glu Cys Lys Asp Trp Phe Leu Gln Ala Pro Lys Arg Lys Ala
    50                  55                  60

Thr Ala Val Leu Gly Pro Pro Arg Lys Gln Cys Pro Cys Asp His Val
65                  70                  75                  80
Lys Gly Arg Glu Lys Lys Asn Arg His Gln Lys His His Arg Lys Ser
                85                  90                  95
Gln Arg Pro Ser Arg Ala Cys Gln Gln Phe Leu Lys Arg Cys His Leu
            100                 105                 110
Ala Ser Phe Ala Leu Pro Leu
        115             119
<210> 4
<211> 357
<212> DNA
<213> Human
<400> 4
ATGAAAGTTC TAATCTCTTC CCTCCTCCTG TTGCTGCCAC TAATGCTGAT GTCCATGGTC  60
TCTAGCAGCC TGAATCCAGG GGTCGCCAGA GGCCACAGGG ACCGAGGCCA GGCTTCTAGG 120
AGATGGCTCC AGGAAGGCGG CCAAGAATGT GAGTGCAAAG ATTGGTTCCT GAGAGCCCCG 180
AGAAGAAAAT TCATGACAGT GTCTGGGCTG CCAAAGAAGC AGTGCCCCTG TGATCATTTC 240
AAGGGCAATG TGAAGAAAAC AAGACACCAA AGGCACCACA GAAAGCCAAA CAAGCATTCC 300
AGAGCCTGCC AGCAATTTCT CAAACAATGT CAGCTAAGAA GCTTTGCTCT GCCTTTG    357
<210> 5
<211> 357
<212> DNA
<213> Rat
<400> 5
ATGAAGCTTC TAGCCTCTCC CTTCCTTCTG TTGCTGACAG GGATGTTCAC GGCCACGGTC  60
TCCAGCAGCC CGAATCAAGA GGTCGCCAGA CACCATGGGG ATCAACACCA GGCTCCTAGG 120
AGGTGGCTCT GGGAAGGTGG CCAAGAGTGT GACTGCAAAG ATTGGTCCCT GCGAGTCTCA 180
AAGAGAAAAA CCACAGCAGT GCTGGAGCCA CCAAGGAAGC AGTGTCCCTG TGATCATGTC 240
AAGGGCAGTG AGAAAAAGAA CAGACGCCAA AAGCACCACA GGAAGTCACA AAGGCCCTCC 300
AGAACCTGCC AGCAATTTCT CAAGCGATGT CAACTAGCAA GCTTCGCCCT GCCCTTA    357
<210> 6

<211> 357
<212> DNA
<213> Mouse
<400> 6
ATGAAGCTTC TAGCCTCTCC CTTCCTTCTG TTGCTTCCAG TGATGCTCAT GTCCATGGTC 60
TTCAGCAGCC CGAACCCAGG GGTCGCCAGA AGCCACGGGG ACCAACACCT GGCTCCTAGG 120
AGGTGGCTCT TGGAAGGTGG CCAAGAATGT GAATGCAAAG ATTGGTTCCT GCAAGCCCCA 180
AAGAGAAAAG CCACAGCAGT GCTGGGGCCA CCAAGGAAGCA GTGTCCCTG TGATCACGTC 240
AAGGGCAGGG AGAAAAAAAA CAGACACCAA AAGCACCACA GGAAGTCGCA AAGACCCTCC 300
AGAGCCTGCC AGCAATTTCT CAAACGATGT CACCTGGCAA GCTTTGCGCT GCCCTTA 357
<210> 7
<211> 97
<212> PRT
<213> Human
<400> 7
Ser Leu Asn Pro Gly Val Ala Arg Gly His Arg Asp Arg Gly Gln Ala
1               5                   10                  15
Ser Arg Arg Trp Leu Gln Glu Gly Gly Gln Glu Cys Glu Cys Lys Asp
            20                  25                  30
Trp Phe Leu Arg Ala Pro Arg Arg Lys Phe Met Thr Val Ser Gly Leu
        35                  40                  45
Pro Lys Lys Gln Cys Pro Cys Asp His Phe Lys Gly Asn Val Lys Lys
    50                  55                  60
Thr Arg His Gln Arg His His Arg Lys Pro Asn Lys His Ser Arg Ala
65                  70                  75                  80
Cys Gln Gln Phe Leu Lys Gln Cys Gln Leu Arg Ser Phe Ala Leu Pro
                85                  90                  95
Leu
97
<210> 8
<211> 97

<212> PRT
<213> Rat
<400> 8
Ser Pro Asn Gln Glu Val Ala Arg His His Gly Asp Gln His Gln Ala
1               5                   10                  15
Pro Arg Arg Trp Leu Trp Glu Gly Gly Gln Glu Cys Asp Cys Lys Asp
            20                  25                  30
Trp Ser Leu Arg Val Ser Lys Arg Lys Thr Thr Ala Val Leu Glu Pro
        35                  40                  45
Pro Arg Lys Gln Cys Pro Cys Asp His Val Lys Gly Ser Glu Lys Lys
    50                  55                  60
Asn Arg Arg Gln Lys His His Arg Lys Ser Gln Arg Pro Ser Arg Thr
65                  70                  75                  80
Cys Gln Gln Phe Leu Lys Arg Cys Gln Leu Ala Ser Phe Ala Leu Pro
                85                  90                  95
Leu
97
<210> 9
<211> 97
<212> PRT
<213> Mouse
<400> 9
Ser Pro Asn Pro Gly Val Ala Arg Ser His Gly Asp Gln His Leu Ala
1               5                   10                  15
Pro Arg Arg Trp Leu Leu Glu Gly Gly Gln Glu Cys Glu Cys Lys Asp
            20                  25                  30
Trp Phe Leu Gln Ala Pro Lys Arg Lys Ala Thr Ala Val Leu Gly Pro
        35                  40                  45
Pro Arg Lys Gln Cys Pro Cys Asp His Val Lys Gly Arg Glu Lys Lys
    50                  55                  60

Asn Arg His Gln Lys His His Arg Lys Ser Gln Arg Pro Ser Arg Ala
65                  70                  75                  80
Cys Gln Gln Phe Leu Lys Arg Cys His Leu Ala Ser Phe Ala Leu Pro
                85                  90                  95
Leu
97
<210> 10
<211> 291
<212> DNA
<213> Human
<400> 10
AGCCTGAATC CAGGGGTCGC CAGAGGCCAC AGGGACCGAG GCCAGGCTTC TAGGAGATGG 60
CTCCAGGAAG GCGGCCAAGA ATGTGAGTGC AAAGATTGGT TCCTGAGAGC CCCGAGAAGA 120
AAATTCATGA CAGTGTCTGG GCTGCCAAAG AAGCAGTGCC CCTGTGATCA TTTCAAGGGC 180
AATGTGAAGA AAACAAGACA CCAAAGGCAC CACAGAAAGC CAAACAAGCA TTCCAGAGCC 240
TGCCAGCAAT TTCTCAAACA ATGTCAGCTA AGAAGCTTTG CTCTGCCTTT G 291
<210> 11
<211> 291
<212> DNA
<213> Rat
<400> 11
AGCCCGAATC AAGAGGTCGC CAGACACCAT GGGGATCAAC ACCAGGCTCC TAGGAGGTGG 60
CTCTGGGAAG GTGGCCAAGA GTGTGACTGC AAAGATTGGT CCCTGCGAGT CTCAAAGAGA 120
AAAACCACAG CAGTGCTGGA GCCACCAAGG AAGCAGTGTC CCTGTGATCA TGTCAAGGGC 180
AGTGAGAAAA AGAACAGACG CCAAAAGCAC CACAGGAAGT CACAAAGGCC CTCCAGAACC 240
TGCCAGCAAT TTCTCAAGCG ATGTCAACTA GCAAGCTTCG CCCTGCCCTT A 291
<210> 12
<211> 291
<212> DNA
<213> Mouse
<400> 12

AGCCCGAACC CAGGGGTCGC CAGAAGCCAC GGGGACCAAC ACCTGGCTCC TAGGAGGTGG 60
CTCTTGGAAG GTGGCCAAGA ATGTGAATGC AAAGATTGGT TCCTGCAAGC CCCAAAGAGA 120
AAAGCCACAG CAGTGCTGGG GCCACCAAGG AAGCAGTGTC CCTGTGATCA CGTCAAGGGC 180
AGGGAGAAAA AAAACAGACA CCAAAAGCAC CACAGGAAGT CGCAAAGACC CTCCAGAGCC 240
TGCCAGCAAT TTCTCAAACG ATGTCACCTG GCAAGCTTTG CGCTGCCCTT A 291
<210> 13
<211> 22
<212> DNA
<213> Artificial Sequence
<220>
<223>
<400> 13
GCCTTTAAGA ACCAACAGAC AG 22
<210> 14
<211> 40
<212> DNA
<213> Artificial Sequence
<220>
<223>
<400> 14
GACGAATTCC CACCATGAAA GTTCTAATCT CTTCCCTCCT 40
<210> 15
<211> 40
<212> DNA
<213> Artificial Sequence
<220>
<223>
<400> 15
GACTCGAGCG GCCGCTACAA AGGCAGAGCA AAGCTTCTTA 40
<210> 16
<211> 47

<212> DNA
<213> Artificial Sequence
<220>
<223>
<400> 16
TGCACCGTCG ACCACCATGA AAGTTCTAAT CTCTTCCCTC CTCCTGT 47
<210> 17
<211> 51
<212> DNA
<213> Artificial Sequence
<220>
<223>
<400> 17
CGCTCAGTCG ACCTACAAAG GCAGAGCAAA GCTTCTTAGC TGACATTGTT T 51
<210> 18
<211> 66
<212> DNA
<213> Artificial Sequence
<220>
<223>
<400> 18
ACAGCAGTCG ACCACCATGA AGCTTCTAGC CTCTCCCTTC CTTCTGTTGC TGACAGGGAT 60
GTTCAC 66
<210> 19
<211> 33
<212> DNA
<213> Artificial Sequence
<220>
<223>
<400> 19
CAGAGTGTCG ACACTATAAG GGCAGGGCGA AGC 33